(19) Europäisches Patentamt
European Patent Office
Office européen des brevets

(11) Veröffentlichungsnummer: **0 237 964**
**A2**

(12) # EUROPÄISCHE PATENTANMELDUNG

(21) Anmeldenummer: **87103590.3**

(22) Anmeldetag: **12.03.87**

(51) Int. Cl.³: **C 07 D 405/14**
C 07 D 413/14, C 07 D 417/14
C 07 D 235/04, C 07 D 263/54
C 07 D 277/62, A 61 K 31/41
A 61 K 31/415, A 61 K 31/42
A 61 K 31/425

(30) Priorität: **21.03.86 DE 3609597**

(43) Veröffentlichungstag der Anmeldung:
**23.09.87 Patentblatt 87/39**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Kampe, Klaus-Dieter, Dr.**
**Am Rehsteig 1**
**D-6232 Bad Soden am Taunus(DE)**

(72) Erfinder: **Raether, Wolfgang, Dr.**
**Falkensteinstrasse 6**
**D-6072 Dreieich(DE)**

(72) Erfinder: **Dittmar, Walter, Dr.**
**Uhlandstrasse 10**
**D-6238 Hofheim am Taunus(DE)**

(54) **2-Azolylmethyl-2-aryl-1,3-dioxolane und deren Salze, Verfahren zu ihrer Herstellung, sie enthaltende Mittel und ihre Verwendung.**

(57) Beschrieben werden Verbindungen der Formel I

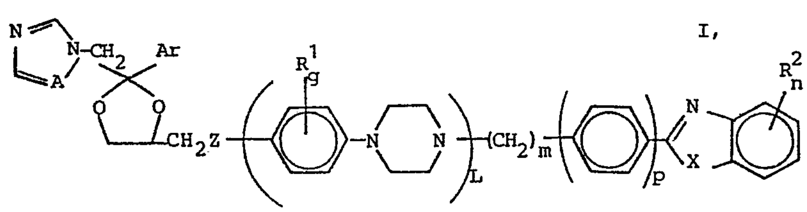
I,

und deren Säureadditionssalze mit A=CH oder N; Ar=Naphthyl, Thienyl, Phenyl; Z=Sauerstoff oder Schwefel; $R^1$=Alkyl, F, Cl; g=0–2; L=0 oder 1; m=0–4; p=0 oder 1; X=0, S oder N-$R^3$; $R^2$=Alkyl, Alkoxy, Halogen, $SCH_3$, $COC_6H_5$, $CF_3$, $COOCH_3$, $COOC_2H_5$, $NO_2$; n=0–2; $R^2$ unter Umständen auch −CH=CH−CH=CH− oder Phenoxy. Beschrieben werden ferner Verfahren zu ihrer Herstellung.

Verbindungen IIIa

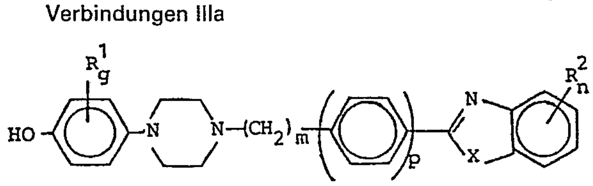
IIIa

sind wertvolle Zwischenprodukte zur Herstellung von I.

Verbindungen I dienen als Antimykotika.

EP 0 237 964 A2

Croydon Printing Company Ltd.

2-Azolylmethyl-2-aryl-1,3-dioxolane und deren Salze, Verfahren zu ihrer Herstellung, sie enthaltende Mittel und ihre Verwendung

Die Erfindung betrifft mit Benzazolyl-Resten substituierte 2-Azolylmethyl-2-aryl-4-[(4-piperazino-phenoxy)-methyl]-1,3-dioxolane einschließlich ihrer Salze, Verfahren zu ihrer Herstellung, diese Verbindungen enthaltende Arzneimittel und ihre Verwendung insbesondere als Antimykotika.

Antimykotisch bzw. fungizid wirksame 2-Azolylmethyl-2-aryl-4-[(4-piperazino-phenoxy)-methyl]-1,3-dioxolane sind bereits bekannt und werden u.a. in der DE-OS 2 804 096, und der EP-OS 7696 beschrieben. Die bekanntesten Vertreter aus der großen Zahl der beschriebenen Verbindungen sind 2-S,(R)-(2,4-Dichlorphenyl)-2-(imidazol-1-ylmethyl)-4-R,(S)-[4-(4-acetyl-piperazin-1-yl)-phenoxymethyl]-1,3-dioxolan (Ketoconazol) und 2-S,(R)-(2,4-Dichlorphenyl)-2-(1,2,4-triazol-1-ylmethyl)-4-R,(S)-[4-(4-isopropyl-piperazin-1-yl)-phenoxymethyl]-1,3-dioxolan (Terconazol), die als Antimykotika im Handel sind (vgl. DOS 2 804 096, Beispiel 20 und Beispiel 53), wobei Ketoconazol vorwiegend als systemisch wirksames und Terconazol als topisch wirksames Antimykotikum verwendet wird. Die antimykotische Wirkung sowie insbesondere die Verträglichkeit der bekannten Verbindungen sind jedoch nicht immer ganz befriedigend.

Es wurde nun gefunden, daß 2-Azolylmethyl-2-aryl-4-[(4-piperazino-phenoxy)-methyl]-1,3-dioxolane der Formel I

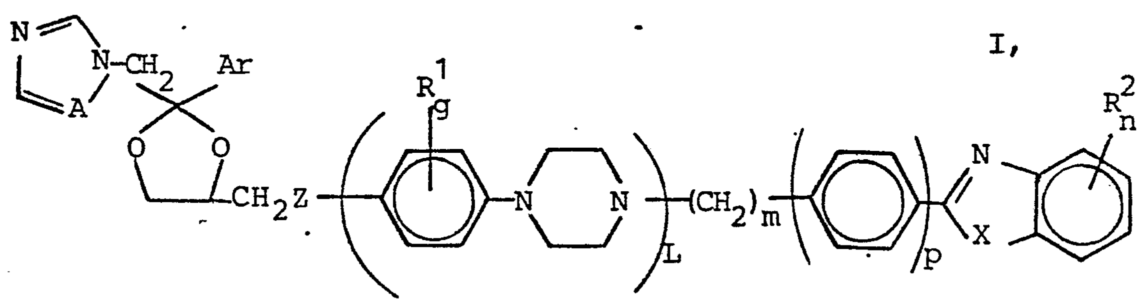

I,

in der bedeuten:

A CH oder N,

Ar Naphthyl, Thienyl, Halothienyl oder eine unsubstituierte oder eine bis zu 3 Substituenten tragende Phenylgruppe
wobei die Substituenten gleich oder verschieden sein können und F, Cl, Br, J, $CF_3$, $OCH_3$, $OC_2H_5$, $CH_3$, $C_2H_5$, $C_6H_5$ oder Phenoxy bedeuten,

Z O oder, falls gleichzeitig L, m und n Null bedeuten, O oder S,

$R^1$ $C_1$-$C_3$-Alkyl, F oder Cl

g 0, 1 oder 2

L O oder 1

m 0, 1, 2, 3 oder 4

p O oder, falls m ungleich O ist oder falls gleichzeitig L und m = O sind, O oder 1 bedeuten und

X O oder,
falls m ungleich O ist oder gleichzeitig L, m und p = O sind oder falls gleichzeitig m und p = O und n ungleich O sind, O, S oder N-$R^3$, wobei

$R^3$ H, $C_1$-$C_4$-Alkyl, Phenyl oder Benzyl bedeutet, bedeuten und

$R^2$ unabhängig voneinander $C_1$-$C_4$-Alkyl, $C_1$-$C_4$-Alkoxy, F, Cl, Br, J, $SCH_3$, CO-$C_6H_5$, $CF_3$, $COOCH_3$, $COOC_2H_5$ oder $NO_2$ und

n 0, 1 oder 2 oder,
falls n=2 ist,

$R^2$ zusätzlich eine -CH=CH-CH=CH-Gruppe bedeutet, die mit dem Phenylring zusammen einen Naphthyl-Rest bildet, oder,
falls gleichzeitig p = O ist und n = 1 ist,

$R^2$ zusätzlich eine unsubstituierte oder im Phenylrest bis zu 2 gleiche oder verschiedene Substituenten tragende Phenoxygruppe darstellt, wobei die Substituenten F, Cl, Br, $OCH_3$, $CH_3$ oder $C_2H_5$ bedeuten,

sowie deren physiologisch verträgliche Säureadditionssalze wertvolle antimykotische bzw. fungizide Eigenschaften besitzen. Sie sind daher zur Bekämpfung von Mykosen bei

Mensch und Tier sowie zur Bekämpfung von Pilzbefall bei Pflanzen und auf Werkstoffen geeignet.

In diesem Zusammenhang ist unter dem Ausdruck "Halothienyl" ein in 2- oder 3-Stellung verknüpfter Thienylrest, der in beliebiger Position mit F, Cl, Br oder J, vorzugsweise Br oder Cl, substituiert sein kann, unter den Ausdrücken "$C_1$-$C_3$- und $C_1$-$C_4$-Alkyl" ein unverzweigter oder verzweigter Kohlenwasserstoffrest mit 1-3 bzw. 1-4 C-Atomen und unter dem Ausdruck "$C_1$-$C_4$-Alkoxy" eine Alkoxygruppe, deren Kohlenwasserstoffrest die unter dem Ausdruck "$C_1$-$C_4$-Alkyl" angegebene Bedeutung hat, zu verstehen.

Bevorzugt sind diejenigen Verbindungen der Formel I, in denen mindestens einer der Substituenten bzw. Indices die folgende Bedeutung hat:

A  CH oder N,
Ar  eine durch 1 oder 2 F- oder Cl-Atome substituierte Phenylgruppe,
Z  O oder,
falls gleichzeitig L, m und p Null bedeuten, O oder S,
$R^1$  $CH_3$ oder $C_2H_5$
g  0 oder 2
L  0 oder 1,
m  0, 1, 2 oder 3,
p  0 oder, falls m = 1 ist, 0 oder 1
X  O, oder
falls m = 1, 2 oder 3 ist, oder falls gleichzeitig L und m = 0 sind, oder falls gleichzeitig m = 0 ist und n = 1 oder 2 ist, O oder S,
$R^2$  unabhängig voneinander $C_1$-$C_4$-n-Alkyl, $C_1$-$C_4$-Alkoxy, F, Cl, Br oder, falls p = 0 und n = 1 ist, $OC_6H_5$ und
n  0, 1 oder 2.

In diesem Zusammenhang ist under dem Ausdruck "$C_1$-$C_4$-n-Alkyl" ein geradkettiger Alkylrest mit 1-4 C-Atomen zu verstehen.

Besonders bevorzugt sind Verbindungen der Formel I, in denen mindestens einer der Substituenten oder Indices die folgende Bedeutung hat:

| | |
|---|---|
| A | CH oder N |
| Ar | 2,4-Dichlorphenyl |
| Z | O |
| $R^1$ | $CH_3$ |
| g | 0 oder 2 |
| L | 0 oder 1 |
| m | 0, 1, 2 oder 3 |
| p | 0 oder, falls m = 1 ist, 0 oder 1 |
| X | O oder, falls m = 1, 2 oder 3 ist oder falls gleichzeitig L und m = 0 sind, O oder S |
| $R^2$ | unabhängig voneinander $CH_3$, $C_2H_5$, $C_1$-$C_4$-Alkoxy, F, Cl oder Br und |
| n | 0, 1 oder 2. |

Besonders bevorzugt sind weiterhin Verbindungen der Formel I, bei denen
gleichzeitig L, m und p Null oder
L Null und gleichzeitig m und p 1 und jeweils X O oder S und n Null oder 1 bedeuten und
$R^2$ unabhängig voneinander $CH_3$, $C_2H_5$, $C_1$-$C_4$-Alkoxy, F, Cl oder Br bedeutet.

Die Erfindung betrifft weiterhin die möglichen Stereoisomeren der Formel I, sowohl als Diastereomeren-Racemate wie auch als reine Enantiomere, sowie ihre pharmazeutisch akzeptablen Salze. Insbesondere betrifft das die wegen der 2,4-Disubstitution des 1,3-Dioxolan-Rings möglichen Stereoisomeren; die 2-Azolylmethyl-Gruppe kann zum 4-ständigen Substituenten, der substituierten Phenoxymethyl-Gruppe, cis- oder trans-ständig angeordnet sein. Die cis-Isomeren zählen zu den bevorzugten erfindungsgemäßen Verbindungen.

Als Salze der erfindungsgemäßen Verbindungen der Formel I kommen solche mit physiologisch unbedenklichen anorganischen und organischen Säuren, wie beispielsweise Chlor-, Brom- oder Jodwasserstoff-, Schwefel-, Phosphor-, Salpeter-, Benzolsulfon-, Toluolsulfon-, Sulfamin-, Methylschwefel-, Essig-, Propion-, Oel-, Palmitin-, Stearin, Malon-, Malein-, Bernstein-, Glutar-, Aepfel-, Wein-, Zitronen-, Fumar-, Milch-, Glykol-, Brenztrauben-, Benzoe-, Toluyl-, Glutamin-, Furancarbon-, Salicyl- oder Mandelsäure in Betracht. Bevorzugt werden Salze mit physiologisch verträglichen anorganischen Säuren, stark bis mittelstark sauren Derivaten solcher Säuren oder mit Fumarsäure.

Von den bekannten, gegen Pilze und Bakterien wirksamen, oben erwähnten Azolylmethyl-2-aryl-4-[(4-piperazino-phenoxy)-methyl]-1,3-dioxolanen unterscheiden sich die erfindungsgemäßen Verbindungen wesentlich durch die Struktur der Substituenten in der 4-Stellung des Dioxolanrings. Von den teilweise ähnlichen, in der EP-A 7 696 erwähnten Verbindungen unterscheiden sich die erfindungsgemäßen Verbindungen entweder durch eine andersartige Struktur des Substituenten in der 4-Stellung des 1,3-Dioxolans oder durch die Art und Stellung der Substituenten an den Benzazol-Einheiten.

Überraschenderweise zeigen die erfindungsgemäßen 2-Azolyl-2-aryl-4-[(4-piperazino-phenoxy)-methyl]-1,3-dioxolane eine breitere und bessere antimykotische Wirkung als die bekannten 2-Azolylmethyl-2-aryl-1,3-dioxolan-Derivate und als das bekannte 2-S,(R)-(2,4-Dichlorphenyl)-2-(imidazol-1-ylmethyl)-4-R,(S)-[4-(4-acetylpiperazin-1-yl)-phenoxymethyl]-1,3-dioxolan (Ketoconazol).

Die Erfindung betrifft weiterhin ein Verfahren zur Herstellung der Verbindungen der Formel I und ihrer Salze, das dadurch gekennzeichnet ist, daß man
A) eine Verbindung der Formel II,

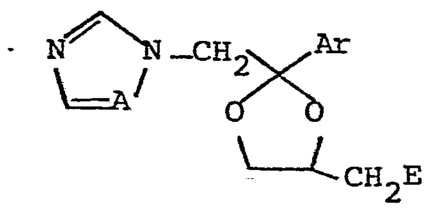

II,

in der

A und Ar die zu Formel I angegebenen Bedeutungen haben und

E Cl, Br, J oder Acyloxy, wie Acetoxy, Trifluoracetyloxy, Benzoyloxy, Nitrobenzoyloxy, $C_1$-$C_3$-Alkylsulfonyloxy wie Methansulfonyloxy, oder Arylsulfonyloxy, wie Benzol-, Nitrobenzol-, Brombenzol- oder Toluolsulfonyloxy, bedeutet,

mit einer Verbindung der Formel III,

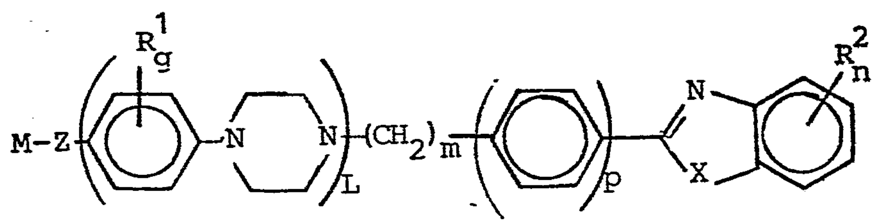

III,

in der

Z O oder, falls gleichzeitig L, m und p Null bedeuten, S bedeutet und

M H, ein Alkali- oder Erdalkalimetall, insbesondere Li, Na oder K, oder $NH_4$ bedeutet und

$R^1$, g, L, m,p, X, $R^2$ und n die zu Formel I angegebenen Bedeutungen haben, umsetzt, oder daß man

B) eine Verbindung der Formel IV,

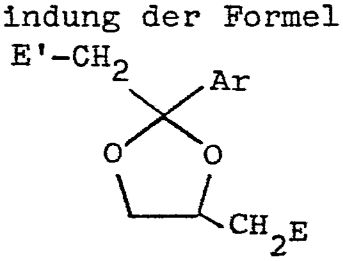

IV

in der
Ar die zu Formel I und E und E' die zu Formel II für E angegebenen Bedeutungen haben,
zunächst mit einer Verbindung der Formel III

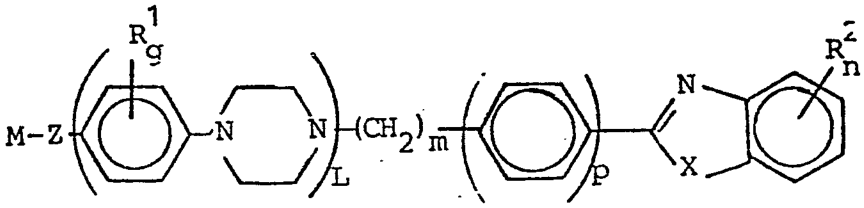

III

umsetzt und hierbei eine Verbindung der Formel V herstellt,

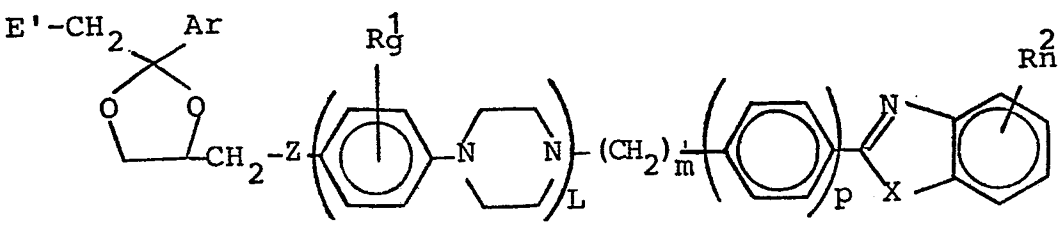

V

in der Z O oder, falls gleichzeitig L, m und p Null bedeuten, S bedeutet, und
Ar, $R^1$, g, L, m, p, X, $R^2$ und n die zu Formel I und E' die zu Formel II für E angegebenen Bedeutungen haben,
und anschließend eine Verbindung der Formel V mit einer Verbindung der Formel VI umsetzt,

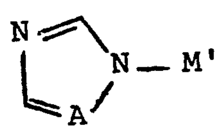

VI

in der
A CH oder N und
M' H, ein Alkali- oder Erdalkalimetall oder $Si(CH_3)_3$ bedeutet,
oder daß man
C) eine Verbindung der Formel VII,

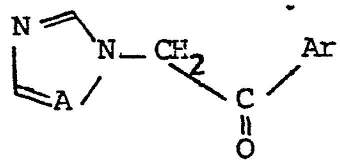

VII

in der A und Ar die zu Formel I angegebenen Bedeutungen haben, mit einem 1,2-Diol der Formel VIII,

0237964

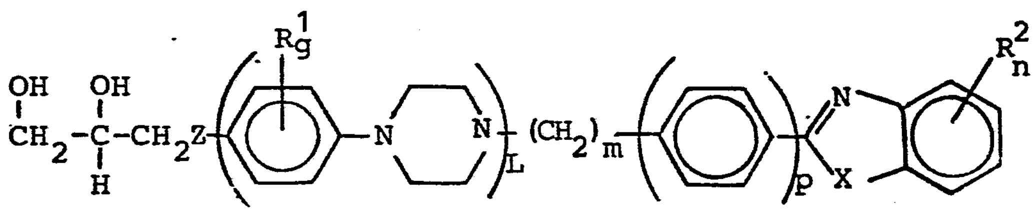

VIII

in der Z, $R^1$, g, L, m, p, X, $R^2$ und n die zu Formel I angegebenen Bedeutungen haben, umsetzt,

oder daß man

D) eine Verbindung der Formel IX,

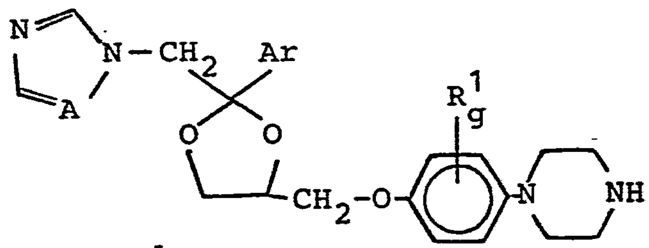

IX

in der A, Ar, $R^1$ und g die zu Formel I angegebenen Bedeutungen haben, oder ein Salz dieser Verbindung, entweder mit einer Verbindung der Formel X,

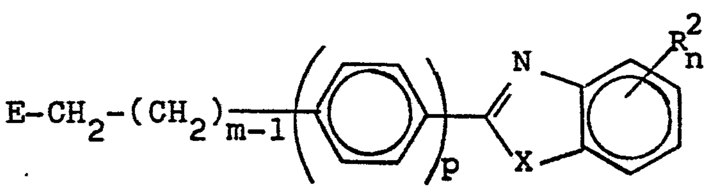

X

in der E Cl, Br, J, Acyloxy, wie Acetyloxy, Trifluoracetyloxy, Benzoyloxy, Nitrobenzoyloxy, $C_1$-$C_3$-Alkylsulfonyloxy, wie Methansulfonyloxy, oder Arylsulfonyloxy, wie Benzol-, Nitrobenzol-, Brombenzol- oder Toluolsulfonyloxy, und

m 1, 2, 3 oder 4 bedeutet, und

p, X, $R^2$ und n die zu Formel I angegebenen Bedeutungen haben,

oder mit einer Verbindung der Formel XI,

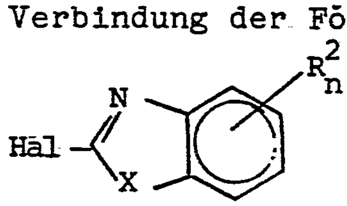

XI

in der

Hal Cl, Br, oder J, vorzugsweise Cl,

X O, oder, falls n ungleich 0 ist, O, S oder $NR^3$, wobei $R^3$ H, $C_1$-$C_4$-Alkyl, Phenyl oder Benzyl bedeutet, bedeuten und

$R^2$ und n die zu Formel I angegebenen Bedeutungen haben, umsetzt, oder daß man

E) eine Verbindung der Formel IIa,

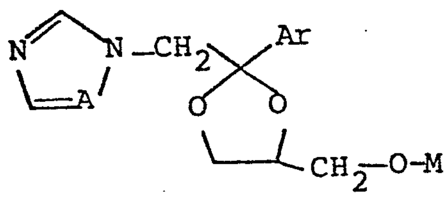 IIa

in der A und Ar die zu Formel I angegebenen Bedeutungen haben und

M H, ein Alkali- oder Erdalkalimetall, insbesondere Li, Na oder K bedeutet,

mit einer Verbindung der Formel X, in der E, m, p, X, $R^2$ und n die zu Formel X angegebenen Bedeutungen haben, unter Bildung einer Verbindung der Formel I, in der Z O bedeutet und A, Ar, $R^1$, g, m, p, X, $R^2$ und n die angegebenen Bedeutungen haben und L = O bedeutet, umsetzt, oder daß man

F) eine Verbindung der Formel IIa,

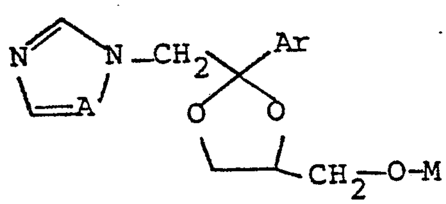 IIa

in der A und Ar die zu Formel I angegebenen Bedeutungen haben und

M H, ein Alkali- oder Erdalkalimetall, insbesondere Li, Na oder K, bedeutet,

mit einer Verbindung der Formel XI,

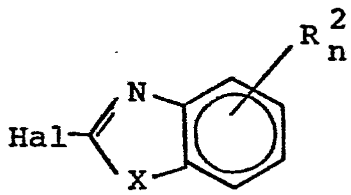

XI

in der

Hal Cl, Br oder J, vorzugsweise Cl, bedeutet und $X$, $R^2$ und n die zu Formel I angegebenen Bedeutungen haben, unter Bildung einer Verbindung der Formel I, in der Z O bedeutet und A, Ar, $R^1$, g, X, $R^2$ und n die angegebenen Bedeutungen haben und L, m und p gleichzeitig O bedeuten, umsetzt

und gegebenenfalls die nach Weg A)-F) erhaltenen Verbindungen der Formel I mit anorganischen oder organischen Säuren in ihre physiologisch verträglichen Salze überführt.

In diesem Zusammenhang ist unter dem Ausdruck "Acyloxy" ein geradkettiger oder verzweigter $C_1$-$C_4$-Alkanoyloxy-Rest, ein Trifluor- oder Trichloracetyl-Rest oder ein im Phenylkern unsubstituierter oder mit bis zu 2 gleichen oder verschiedenen Substituenten substituierter Benzoyloxy-Rest zu verstehen, wobei die Substituenten $CH_3$, $OCH_3$, F, Cl, Br oder $CH_3$ bedeuten können, unter dem Ausdruck "Arylsulfonyloxy" ein unsubstituierter oder ein mit einem Cl, Br, $CH_3$, $C_2H_5$, $OCH_3$, $OC_2H_5$ oder $NO_2$ substituierter Phenylsulfonyloxy- oder ein Naphthylsulfonyloxy-Rest zu verstehen und unter dem Ausdruck "$C_1$-$C_3$-Alkylsulfonyloxy" ein n-Alkansulfonsäure-Rest mit 1-3 C-Atomen zu verstehen. Der Ausdruck "Alkalimetall" soll Li, Na oder K bedeuten.

Die Verfahrens-Variante A), wobei bei den Verbindungen der Formel II E bevorzugt Cl, Br, Acetoxy, Trifluoracetoxy, Methansulfonyloxy oder (subst.) Phenylsulfonyloxy bedeutet und bei den Verbindungen der Formel III M, Z, $R^1$, g, p, X, $R^2$ und n die angegebenen Bedeutungen haben, und bevorzugt L 1, m O, 1, 2 oder 3 bedeuten, oder bevorzugt L

und m gleichzeitig 0 oder L, m und p gleichzeitig 0 bedeuten, wird bei einer Temperatur zwischen 20°C und 150°C, vorteilhaft zwischen 40°C und 110°C, in Anwesenheit einer Base und zweckmäßig in einem inerten organischen Lösungsmittel, wie beispielsweise N,N-Dimethylformamid, N,N-Dimethylacetamid, Dimethylsulfoxid, N-Methylpyrrolidon-2, 1,2-Dimethoxyethan, Dioxan, Tetrahydrofuran, 4-Methyl-2-pentanon, Methanol, Ethanol, Isopropylalkohol, Propanol, Butanol, Pentanol, tert.-Butylalkohol, Methylglykol, Methylenchlorid, Acetonitril oder einem aromatischen Kohlenwasserstoff wie Benzol, Chlorbenzol, Toluol oder Xylol durchgeführt. Es können auch Gemische der beispielhaft genannten Lösungsmittel verwendet werden.

Geeignete Basen sind beispielsweise Alkalimetall- oder Erdalkalimetall-carbonate, -hydrogencarbonate, -hydroxide, -amide, -alkoholate oder -hydride wie z.B. Natriumcarbonat, Natriumhydrogencarbonat, Kaliumcarbonat, Natriumhydroxid, -amid oder -methylat, Kalium-t-butylat oder Natriumhydrid, oder organische Basen, z.B. tertiäre Amine wie Triethylamin, Tributylamin, Ethylmorpholin oder Pyridin, Dimethylaminopyridin, Chinolin oder 1,5-Diazabicyclo-[5,4,0]undec-5-en (DBU).

Die Umsetzung kann ebenso unter den Bedingungen einer Phasentransferreaktion ausgeführt werden, indem man die Reaktionspartner in einem geeigneten Lösungsmittel, wie z.B. Ether, Dioxan, Tetrahydrofuran, Methylenchlorid, N,N-Dimethylform- oder -acetamid, N-Methylpyrrolidon-(2), Butanol, tert.-Butanol, einem aliphatischen, cycloaliphatischen oder aromatischen Kohlenwasserstoff wie Hexan, Cyclohexan, Benzol, Toluol oder Xylol, Methylglykol, Anisol oder Chlorbenzol unter kräftigem Rühren in Gegenwart eines Phasentransferkatalysators und entweder einem gepulverten Alkalihydroxid, wie z. B. Natrium- oder Kaliumhydroxid oder einer konzentrierten wäßrigen Lösung derselben, vorzugsweise in einem Temperaturbereich von 20°C bis 120°C aufeinander einwirken läßt.

Geeignete Phasentransferkatalysatoren sind z.B. Trialkylbenzylammonium- oder Tetraalkylammonium-halogenide, -hydroxide, -hydrogensulfate mit vorzugsweise 1 bis 12 C-Atomen im Alkylrest oder Kronenether, wie z.B. 12-Crown-4, 15-Crown-5, 18-Crown-6 oder Dibenzo-18-crown-6.

Herstellung der Ausgangsstoffe:

Die Ausgangsverbindungen der Formel II, in der Ar und A die zu Formel I angegebenen Bedeutungen haben, sind teilweise bekannt; die nicht bekannten können in Analogie zu den bekannten hergestellt werden.

Die Ausgangsverbindungen der Formel III, worin L und m = 0 und p = 1 bedeuten, sind teils bekannt oder können analog den bekannten Verbindungen hergestellt werden. Diejenigen, worin L = 0, m = 1,2 oder 3, bevorzugt 1, und p = 0 oder 1 bedeuten, können aus den entsprechenden bekannten Chlor- oder Bromverbindungen der Formeln Xa

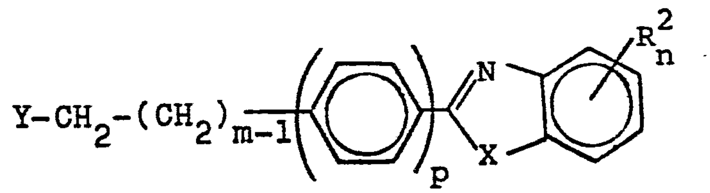

Xa

(Y=Cl oder Br) durch alkalische Hydrolyse hergestellt werden. Diejenigen Verbindungen der Formel III, worin L, m und p gleichzeitig 0 und Z S bedeuten, sind z. T. bekannt oder können nach bekannten Methoden, bei bestimmten Vertretern beispielsweise ausgehend von entsprechenden 2-Chloranilinen und Natrium-Ethylxanthat, hergestellt werden.

Die Verfahrens-Variante B), wobei eine Verbindung der Formel IV, in der E bevorzugt Br, J oder Trifluoracetyloxy, Methansulfonyloxy, Benzol-, Nitrobenzol-, Brombenzol- oder Toluolsulfonyloxy und E' bevorzugt Cl oder Br bedeuten, mit einer Verbindung der Formel III, in der M, Z, $R^1$, g, L, m, p, X, $R^2$ und n die angegebenen Bedeutungen haben,

und bevorzugt L = 1 oder gleichzeitig L und m oder gleichzeitig L, m und p Null bedeuten, unter Bildung einer Verbindung der Formel V umgesetzt wird, wird unter den gleichen Reaktionsbedingungen wie bei Variante A zur Herstellung von Verbindungen der Formel I angegeben durchgeführt.

Die Herstellung von Verbindungen der Formel I durch Umsetzung von Verbindungen der Formel V mit Verbindungen der Formel VI erfolgt zweckmäßig in einem inerten Lösungsmittel in Gegenwart einer Base, wie zum ersten Herstellverfahren vorstehend angegeben, vorzugsweise in einem Temperaturbereich von 100 bis 190°C. Die Umsetzung erfolgt zweckmäßigerweise in Anwesenheit eines Alkalijodides wie z.B. Natrium- oder Kaliumjodid, gegebenenfalls in einem Autoklaven unter Druck.

Die vorstehend beschriebenen Umsetzungen können zweckmäßig als Eintopfreaktion durchgeführt werden, indem man zunächst bei 40 bis 100°C eine Verbindung der Formel IV mit einer Verbindung der Formel III in Gegenwart einer Base in einem inerten Lösungsmittel umsetzt. Anschliessend wird ohne Isolierung der Verbindung der Formel V eine Verbindung der Formel VI und gegebenenfalls ein weiteres Moläquivalent einer Base und ein Alkalijodid (z.B. Kaliumjodid) zugegeben und auf 100 bis 190°C erhitzt.

Herstellung der Ausgangsstoffe:

Verbindungen der Formel IV, in der E und E' die zur Formel II für E angegebenen Bedeutungen haben, sind bekannt. Sie werden hergestellt, indem man eine Verbindung der Formel XII in üblicher Weise in eine reaktionsfähige Estergruppe überführt. So werden z.B. die Verbindungen der Formel IV, in der E' bevorzugt Cl oder Br und E Methansulfonyloxy bedeuten, hergestellt, indem man eine Verbindung der Formel

XII in der Ar die zu Formel I angegebenen Bedeutungen hat und E' Cl oder Br bedeuten, mit Methansulfochlorid bei -10°C bis +50°C zweckmäßig in einem inerten Lösungsmittel, in Gegenwart einer Base umsetzt. Verbindungen der Formel IV, in der E beispielsweise Brom bedeutet, werden durch Umsetzung von Verbindungen der Formel XII

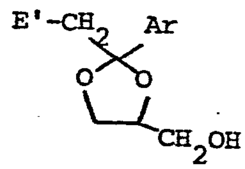

XII

(E' = Cl oder Br) mit Bromierungsmitteln, wie z.B. $PBr_3$ oder $POBr_3$ in einem inerten Lösungsmittel bei 0°C bis 100°C hergestellt. Derartige Verbindungen können auch hergestellt werden, indem man eine Verbindung der Formel XIII,

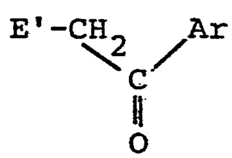

XIII

in der E' Cl oder Br bedeutet und Ar die angegebenen Bedeutungen hat, mit 1-Brom-2,3-propandiol in einem inerten Lösungsmittel in Gegenwart einer starken Säure unter Bildung eines 1,3-Dioxolans nach für derartige Ketalisierungen bekannten Methoden umsetzt.
Die Verbindungen der Formel XII sind bekannt.

Die Verfahrens-Variante C), wobei man eine Verbindung der Formel VII mit einer Verbindung der Formel VIII unter Bildung einer Verbindung der Formel I umsetzt, wird im allgemeinen unter den gleichen Bedingungen durchgeführt, wie die Herstellung von Verbindungen der Formel IV (Variante B). Die Ketalisierung von Ketonen der Formel VII mit Glycerin-Derivaten der Formel VIII erfolgt vorteilhaft in einem Lösungsmittelgemisch, bestehend aus einem inerten Lösungsmittel, das mit Wasser ein azeotropes Gemisch bildet, wie z.B. Benzol, Toluol, Xylol, Chlorbenzol oder Cyclohexan, und einem Alkohol, in Anwesenheit einer starken Säure in einem Temperaturbereich von 75 bis 180°C. Vorteilhaft werden bei dieser Ketalisierung mindestens 1,5 Äquivalente einer starken Säure (bez. auf die Azolverbindung der Formel VII) und als Alkohole aliphatische Alkohole mit einem Kp zwischen 75° und 150°C und/oder Monoether von niederen Diolen, die zwischen 100° und 150°C sieden, verwendet.

Herstellung der Ausgangsstoffe:

Die Verbindungen der Formel VII sind bekannt und können nach beschriebenen Methoden hergestellt werden.

Verbindungen der Formel VIII, in der $R^1$, g, L, m, p, X, $R^2$ und n die zu Formel III angegebenen Bedeutungen haben und L, m und p nicht gleichzeitig 0 bedeuten und in der bevorzugt L und m gleichzeitig 0 bedeuten, werden durch Umsetzung von Verbindungen der Formel III mit 1-Halogen-2,3-propandiol hergestellt, in analoger Weise wie in Org. Synth. Collect. Vol. I, S. 296 beschrieben. In diesem Zusammenhang bedeutet Halogen Cl, Br oder J. Verbindungen der Formel VIII, in der L, m und p gleichzeitig 0 bedeuten und X, $R^2$ und n die angegebenen Bedeutungen haben, werden durch Umsetzung von Glycerin mit 2-Chlorbenzazolen der Formel XIa

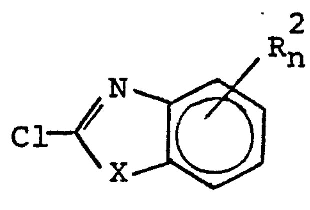

XIa

in Gegenwart von 1 Äquivalent einer Base, zweckmäßig in einem inerten Lösungsmittel, hergestellt. Diejenigen Verbindungen der Formel VIII, worin Z S und L, m und p gleichzeitig 0 bedeuten, werden durch Umsetzung von 1-Halogen-2,3-propandiolen mit Verbindungen der Formel III, in denen Z S und L, m und p gleichzeitig 0 bedeuten,

vorteilhaft in Gegenwart von bis zu einem Äquivalent einer Base, zweckmäßig in einem inerten Lösungsmittel, hergestellt.

Bei der Verfahrensvariante D) wird eine Verbindung der Formel IX entweder mit einer Verbindung der Formel X, in der E, m, p, X, $R^2$ und n die angegebenen Bedeutungen haben, und, falls p = 1 ist, m bevorzugt 1 bedeutet, oder mit einer Verbindung der Formel XI, in der Hal, X, $R^2$ und n die angegebenen Bedeutungen haben, zweckmäßig in einem inerten organischen Lösungsmittel in einem Temperaturbereich von 0° bis 180°C, vorzugsweise von 30° bis 120°C, umgesetzt. Diese Umsetzung wird vorteilhaft in Gegenwart einer Base, die vorzugsweise in äquimolarer Menge angewandt wird, durchgeführt.

Die Synthese von Verbindungen der Formel I aus den Verbindungen der Formeln IX, X oder XI kann auch ohne Basenzusatz ausgeführt werden. Die Reaktanten der Formeln IX und X oder XI können in unterschiedlichen Molverhältnissen angewendet werden, d.h. es können entweder jeweils die Verbindungen der Formel IX oder diejenigen der Formeln X oder XI im Überschuß angewandt werden, vorteilthaft werden äquimolare Mengen angewendet.

Als Lösungsmittel kommen beispielsweise Kohlenwasserstoffe, allgemein Ether wie Diethylether, 1,2-Dimethoxyethan, Tetrahydrofuran oder Dioxan, oder Acetonitril, Butyronitril, Dimethylform- oder -acetamid, Aceton, 4-Methyl-2-pentanon, Methylenchlorid, Chloroform, Dimethylsulfoxid, Anisol, Chlorbenzol, Tetrachlorethen oder Gemische dieser Lösungsmittel in Frage.

Geeignete Basen sind die beispielhaft bei der Verfahrens-Variante A) erläuterten.

Die Umsetzung kann ebenso unter den Bedingungen einer Phasentransferreaktion, wie sie bei der Beschreibung der Verfahrens-Variante A) erläutert sind, ausgeführt werden.

Herstellung der Ausgangsstoffe:

Die Verbindungen der Formel IX sind z.T. bekannt (vgl. DOS 2 804 096, z.B. Beispiel 21); solche, die von den bekannten abweichende Bedeutungen für Ar haben und/oder bei denen g 1 oder 2 bedeutet, können in Analogie zu den bekannten (vgl. DOS 2 804 096) hergestellt werden.

Die Verbindungen der Formel X, in der E, m, p, X, $R^2$ und n die angegebenen Bedeutungen haben, sind teilweise bekannt. Das trifft vor allem für Verbindungen der Formel X mit E = Cl zu.

Bevorzugt bedeuten bei den Verbindungen der Formel X E = Cl und, falls p = 1 ist, m = 1. Die Verbindungen der Formel X mit E = Cl oder Brom werden hergestellt, indem man ein Säurechlorid der Formel XIV,

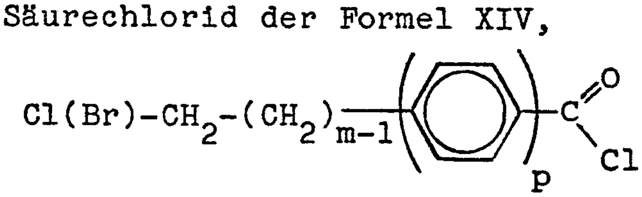

XIV

in der m und p die angegebenenen Bedeutungen haben, mit einer Verbindung der Formel XV,

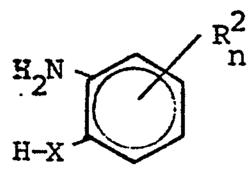

XV

in der X, $R^2$ und n die zu Formel X angegebenen Bedeutungen haben, zweckmäßig in einem inerten organischen Lösungsmittel in einem Temperaturbereich von 40 - 180°C umsetzt. Vorteilhaft wird dabei in einem oberhalb 60°C siedenden inerten Lösungsmittel gearbeitet, das mit Wasser ein azeotropes Gemisch bildet und somit zur Entfernung von gebildetem Wasser aus der Reaktionsmischung geeignet ist, wie beispielsweise Benzol, Chlorbenzol oder Toluol.

 

Die Verbindungen der Formel XI, in der Hal, X, $R^2$ und n die angegebenen Bedeutungen haben, sind entweder bekannt oder können nach bekannten Methoden hergestellt werden.

Die Verfahrensvariante E), wobei man eine Verbindung der Formel IIa mit einer der Formel X umsetzt, wird zweckmäßig in einem inerten, aprotischen Lösungsmittel in einem Temperaturbereich von 30-150°C, vorteilhaft in einem wasserfreien Medium in Gegenwart einer Base, die vorzugsweise in äquimolarer Menge angewandt wird, durchgeführt.

Geeignete Lösungsmittel sind beispielsweise:

N,N-Dimethylformamid, N,N-Dimethylacetamid, Dimethylsulfoxid, N-Methylpyrrolidon-2, Dioxan, Tetrahydrofuran, 1,2-Dimethoxyethan, 4-Methyl-2-pentanon oder Acetonitril.

Es können auch Gemische der beispielhaft genannten Lösungsmittel verwendet werden.

Geeignete Basen sind beispielsweise Alkalimetall-amide, -carbonate, -hydroxide, -alkoholate oder -hydride wie Natriumcarbonat, Kaliumcarbonat, Natriumhydroxid, Natriummethylat, Kalium-t.-butylat, Natriumhydrid oder Natriumamid, oder organische Basen, z.B. tertiäre Amine wie Triethylamin, Tributylamin, Ethylmorpholin oder Dimethylaminopyridin oder 1,5-Diazabicyclo[5,4,0]undec-5-en (DBU).

Die Umsetzung kann ebenso unter den Bedingungen einer Phasentransferreaktion ausgeführt werden, indem man die Reaktionspartner in einem geeigneten Lösungsmittel, wie Ether, Dioxan, Tetrahydrofuran, Methylenchlorid, N,N-Dimethylform- oder -acetamid, N-Methylpyrrolidon-(2), einem aliphatischen, cycloaliphatischen oder aromatischen Kohlenwasserstoff wie Hexan, Cyclohexan, Benzol, Toluol oder Xylol, Anisol oder Chlorbenzol unter kräftigem Rühren in Gegenwart eines Phasentransferkatalysators und entweder

einem gepulverten Alkalihydroxid, wie Natrium- oder Kaliumhydroxid oder einer konzentrierten wäßrigen Lösung derselben, vorzugsweise in einem Temperaturbereich von 40° bis 150°C aufeinander einwirken läßt.

Geeignete Phasentransferkatalysatoren sind z.B. Trialkylbenzylammonium- oder Tetraalkylammonium-halogenide, -hydroxide, -hydrogensulfate mit vorzugsweise 1 bis 12 C-Atomen im Alkylrest oder Kronenether, wie z.B. 12-Crown-4, 15-Crown-5, 18-Crown-6 oder Dibenzo-18-crown-6.

Die Verfahrensvariante F), wobei eine Verbindung der Formel IIa mit einer der Formel XI unter Bildung einer Verbindung der Formel I, in der Z O bedeutet und A, Ar, $R^1$, g, X, $R^2$ und n die angegebenen Bedeutungen haben und L, m und p O bedeuten, umgesetzt wird, wird unter den gleichen Bedingungen, eingeschlossen diejenigen einer Phasentransferreaktion, wie bei der Verfahrensvariante E) beschrieben, durchgeführt.

Die Verbindungen der Formel IIa und XI sind bekannt oder können in Analogie zu den bekannten hergestellt werden. Ebenso erfolgt die Bildung von Alkali- oder Erdalkali-Alkoholaten der Formel IIa, worin M ein solches Metallatom bedeutet, nach bekannten Methoden.

Bevorzugt werden zur Herstellung von Verbindungen der Formel I die Verfahrens-Varianten A, B, D, E und F angewendet.

Die Reaktionszeiten betragen je nach Verfahrensvariante und je nach Temperaturbereich wenige Minuten bis einige Stunden.

Falls erforderlich, kann eine Reinigung der Verfahrenserzeugnisse durch Umkristallisation aus einem geeigneten Lösungsmittel oder Lösungsmittelgemisch oder durch Säulenchromatographie an Kieselgel erfolgen.

Die diastereomeren Racemate (cis- oder trans-Form) der

Verbindungen der Formel I können in üblicher Weise, z.B. durch selektive, fraktionierte Kristallisation oder Säulenchromatographie getrennt werden.

Da die stereochemische Konfiguration bereits in den Zwischenprodukten der Formel II vorgegeben ist, kann die Trennung in die cis- und trans-Form bereits auf dieser Stufe oder noch früher, beispielsweise auf der Stufe der Zwischenprodukte der allgemeinen Formel IV, oder bei den Zwischenprodukten der Formel IX erfolgen.

Die cis- und trans-diastereomeren Racemate können ihrerseits in üblicher Weise in ihre optischen Antipoden cis(+), cis(-) bzw. trans(+) und trans(-) getrennt werden.

Bevorzugt werden die Verbindungen I nach den Verfahrensvarianten A, B, D, E und F hergestellt.

Die Erfindung betrifft weiterhin Verbindungen der Formel IIIa,

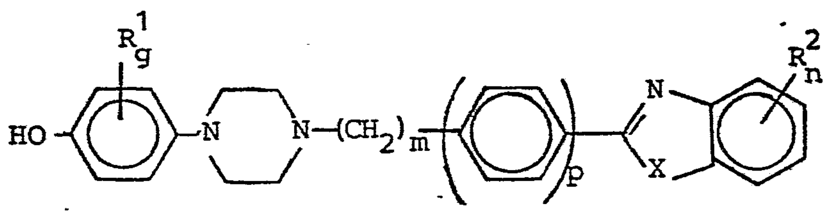

IIIa,

in der bedeuten:

$R^1$ $C_1$-$C_3$-Alkyl, F oder Cl,

g 0, 1 oder 2,

m 0, 1, 2, 3 oder 4,

p 0 oder,
falls m ungleich 0 ist, 0 oder 1 und

X 0 oder,
falls m ungleich 0 ist oder falls gleichzeitig m und p = 0 und n ungleich 0 sind, O, S oder N-$R^3$, wobei

$R^3$ H, $C_1$-$C_4$-Alkyl, Phenyl oder Benzyl bedeutet, und

$R^2$ unabhängig voneinander $C_1$-$C_4$-Alkyl, $C_1$-$C_4$-Alkoxy, F, Cl, Br, J, $SCH_3$, CO-$C_6H_5$, $CF_3$ oder $NO_2$ und

n 0, 1 oder 2

oder, falls n = 2 ist,

$R^2$ zusätzlich eine $-CH{=}CH-CH{=}CH-$Gruppe, die mit dem Phenylring zusammen einen Naphthyl-Rest bildet,

oder, falls p = 0 ist und n = 1 ist,

$R^2$ zusätzlich eine unsubstituierte oder im Phenylrest bis zu 2 gleiche oder verschiedene Substituenten tragende Phenoxygruppe, wobei die Substituenten F, Cl, Br, $OCH_3$, $CH_3$ oder $C_2H_5$ bedeuten,

sowie deren Säureadditionssalze.

Bevorzugt werden diejenigen Verbindungen der Formel IIIa, in denen mindestens einer der Substituenten bzw. Indizes die folgende Bedeutung hat:

$R^1$ $CH_3$ oder $C_2H_5$

g Null oder 2,

m Null, 1, 2 oder 3,

p Null oder, falls m ungleich Null ist, Null oder 1,

X O oder, falls m = 1, 2 oder 3 ist, oder falls m = Null ist und n =1 oder 2 ist, O oder S,

$R^2$ unabhängig voneinander $C_1$-$C_4$-n-Alkyl, $OCH_3$, $OC_2H_5$, F, Cl, Br oder, falls gleichzeitig p = 0 und n = 1 ist, $OC_6H_5$ und

n Null, 1 oder 2 und, falls p = 1 ist, bedeutet m vorzugsweise 1.

In diesem Zusammenhang ist unter dem Ausdruck "$C_1$-$C_4$-n-Alkyl" ein geradkettiger Alkylrest mit 1-4 C-Atomen zu verstehen.

Besonders bevorzugt sind Verbindungen der Formel IIIa, in denen mindestens einer der Substituenten bzw. Indizes die folgende Bedeutung hat:

$R^1$ $CH_3$

g Null oder 2,

m Null, 1, 2 oder 3,

p Null oder, falls m ungleich Null ist, Null oder 1

X O oder, falls m = 1, 2 oder 3 ist, O oder S

 0237964

$R^2$ unabhängig voneinander $CH_3$, $C_2H_5$, $OCH_3$, $OC_2H_5$, Cl oder Br und

n Null, 1 oder 2 und

falls p = 1 ist, m vorzugsweise 1 bedeutet.

Die Verbindungen der Formel IIIa, in denen $R^1$, g, m, p, X, $R^2$ und n die angegebenen Bedeutungen haben, sind neu und stellen wertvolle Zwischenprodukte zur Herstellung der stark antimykotisch sowie fungizid wirkenden Verbindungen der Formel I dar. Ein Teil der Verbindungen der Formel IIIa wirkt ebenfalls antimykotisch sowie fungizid. Ein Teil der Verbindungen der Formel IIIa zeigt darüber hinaus Wirkungen auf das Herz-Kreislauf-System.

Die Erfindung betrifft außerdem ein Verfahren zur Herstellung von Verbindungen der Formel IIIa, das dadurch gekennzeichnet ist, daß man eine Verbindung der Formel XVI,

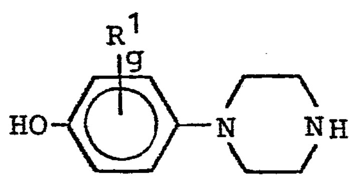

XVI

in der $R^1$ und g die zu Formel IIIa angegebenen Bedeutungen haben, oder ein Salz dieser Verbindung, entweder mit einer Verbindung der Formel X,

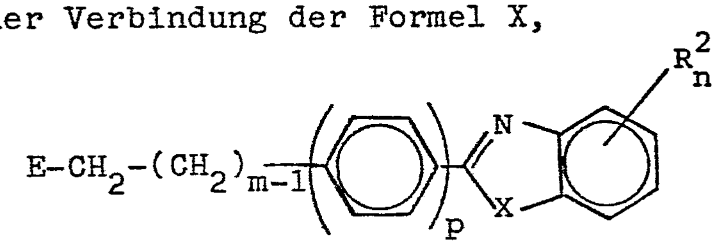

X

in der

E Cl, Br, J, Acyloxy, wie Acetoxy, Trifluoracetoxy, Benzoyloxy, Nitrobenzoyloxy, $C_1$-$C_3$-Alkylsulfonyloxy, wie Methansulfonyloxy, oder Arylsulfonyloxy, wie Benzol-, Nitrobenzol-, Brombenzol- oder Toluolsulfonyloxy und

m 1, 2, 3 oder 4 bedeutet, und

p, X, $R^2$ und n die zu Formel IIIa angegebenen Bedeutungen haben,

oder mit einer Verbindung der Formel XI,

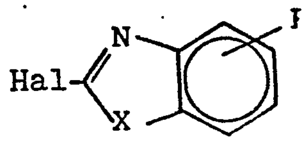

XI

in der

Hal Cl, Br oder J, vorzugsweise Cl,

X O, oder falls n ungleich Null ist, O, S oder $N-R^3$, wobei $R^3$ H, $C_1$-$C_4$-Alkyl, Phenyl oder Benzyl bedeutet, bedeuten und

$R^2$ und n die zu Formel IIIa angegebenen Bedeutungen haben, umsetzt,

und gegebenenfalls die erhaltenen Verbindungen der Formel IIIa mit anorganischen oder organischen Säuren in ihre Säureadditionssalze überführt.

Bevorzugt wird für die erfindungsgemäße Umsetzung mit einer Verbindung der Formel XVI entweder

eine Verbindung der Formel X, in der

E Cl, Br oder J,

m 1, 2 oder 3,

p Null oder 1,

X O oder S,

$R^2$ unabhängig voneinander $C_1$-$C_4$-n-Alkyl, $OCH_3$, $OC_2H_5$, F, Cl, Br oder, falls p = Null und n = 1 ist, $OC_6H_5$ und

n Null, 1 oder 2 bedeuten, oder

eine Verbindung der Formel XI, in der

Hal Cl,

X O oder, falls n ungleich Null ist, O oder S,

$R^2$ unabhängig voneinander $C_1$-$C_4$-n-Alkyl, $OCH_3$, $OC_2H_5$, F, Cl, Br oder, falls n = 1 ist, $OC_6H_5$ und

n Null, 1 oder 2 bedeuten.

Das erfindungsgemäße Verfahren zur Herstellung von Verbindungen der Formel IIIa wird zweckmäßig in einem inerten organischen Lösungsmittel in einem Temperaturbereich von

0° bis 180°C, vorzugsweise von 30° bis 120°C, vorteilhaft in Gegenwart einer Base, die vorzugsweise in äquivalenter Menge angewandt wird, durchgeführt. Werden für das Verfahren Salze der Verbindungen der Formel XVI verwandt, dann wird die dem Salzanteil entsprechende stöchiometrische Menge an Base zugesetzt. Wahlweise kann darüber hinaus dann ein weiterer Anteil an Base angewandt werden.

Die Synthese von Verbindungen der Formel IIIa aus den Verbindungen der Formeln XVI und X oder XI kann, sofern die Verbindungen XVI nicht als Salz angewandt werden, auch ohne Basenzusatz ausgeführt werden. Die Reaktanten der Formeln XVI und X oder XI können in unterschiedlichen Molverhältnissen angewandt werden, d.h. es können entweder jeweils die Verbindungen der Formel XVI oder diejenigen der Formel X oder XI im Überschuß angewandt werden, vorteilhaft werden äquimolare Mengen angewandt.

Als Lösungsmittel kommen beispielsweise Kohlenwasserstoffe, allgemein Ether, wie Diethylether, 1,2-Dimethoxyethan, Tetrahydrofuran oder Dioxan, oder Acetonitril, Butyronitril, Dimethylform- oder -acetamid, Aceton, 4-Methyl-2-pentanon, Methylenchlorid, Chloroform, Dimethylsulfoxid, Anisol, Chlorbenzol, Tetrachlorethen oder Gemische dieser Lösungsmittel in Frage.

Geeignete Basen sind die beispielhaft bei der Verfahrens-Variante A) erläuterten.

Die Reaktionszeiten betragen je nach Temperaturbereich wenige Minuten bis einige Stunden.

Falls erforderlich, kann eine Reinigung der Verfahrenserzeugnisse durch Umkristallisation aus einem geeigneten Lösungsmittel oder Lösungsmittelgemisch oder durch Säulenchromatographie an Kieselgel erfolgen.

Herstellung der Ausgangsstoffe:

Die Verbindung der Formel XVI mit g=0 ist bekannt. Verbindungen der Formel XVI, bei denen g 1 oder 2 bedeutet und $R^1$ die zu Formel I angegebenen Bedeutungen hat, werden in Analogie zu den bekannten Verbindungen durch Umsetzung von entsprechenden 4-Methoxy-Anilinen mit Bis-(2-Chlorethyl)-amin und anschließender Phenolether-Spaltung mit konz. wäßriger Bromwasserstoffsäure hergestellt. Die Herstellung der Verbindungen der Formeln X und XI, in denen E , m, p, X, $R^2$, n und Hal die angegebenen Bedeutungen haben, ist bereits bei der Herstellung der Ausgangsstoffe für die Verfahrensvariante D) zur Herstellung von Verbindungen der Formel I beschrieben.

Die Verbindungen der Formel I und ihre Säureadditionssalze sind wertvolle Arzneimittel. Sie wirken antimikrobiell und eignen sich insbesondere zur Vorbeugung und Behandlung von Pilzinfektionen beim Menschen und bei verschiedenen Säugetierarten.

Die neuen Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Diejenigen Verbindungen der Formel I, worin L, m und p Null und Z O oder S bedeuten und A, Ar, X, $R^2$ und n die angegebenen Bedeutungen haben, sind besonders für lokale Anwendungen geeignet, während solche, bei denen L, m und p, insbesondere L und m, im Rahmen der angegebenen Bedeutungen ungleich Null sind, sowohl für orale und parenterale als auch für lokale Anwendungen verwendet werden können.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als Darreichungsformen kommen beispielsweise Tabletten, Dragees, Kapseln, Pillen, wäßrige Lösungen, Suspensionen

und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die therapeutisch wirksamen Verbindungen sollen in den oben aufgeführten pharmazeutischen Zubereitungen zweckmäßig in einer Konzentration von etwa 0,01 bis 99,0, vorzugsweise von etwa 0,05 bis 50 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,05 bis etwa 200, vorzugsweise 0,1 bis 100, insbesondere 0,5 bis 30 mg/kg Körpergewicht je 24 Stunden, gegebenenfalls in Form mehrerer Einzelgaben zur Erzielung der gewünschten Ergebnisse zu verabreichen. Die Gesamtmenge wird

in 1 bis 8, vorzugsweise in 1 bis 3 Einzeldosen verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen der Formel I eignen sich auch zur Behandlung von Protozoenerkrankungen beim Menschen und Tier wie sie z. B. durch Infektionen mit Trichomonas vaginalis und Entamoeba histolytica sowie durch Trypanosoma cruzi, Leishmania donovani hervorgerufen werden.

Die neuen Verbindungen können oral oder lokal angewendet werden. Die orale Anwendung erfolgt in pharmazeutisch üblichen Zubereitungen, z. B. in Form von Tabletten oder Kapseln.

Die neuen Verbindungen der allgemeinen Formel I sind auch als Biozide wirksam. Sie zeichnen sich insbesondere durch ihre fungizide Wirksamkeit bei phytopathogenen Pilzen aus. Selbst bereits in das pflanzliche Gewebe eingedrungene pilzliche Krankheitserreger lassen sich erfolgreich bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der neuen Verbindungen erfaßt eine Vielzahl verschiedener phytopathogener Pilze, wie z.B. Piricularia oryzae, Plasmopara viticola, verschiedene Rostarten, vor allem aber Venturia inaequalis, Cercospora beticola und echte Mehltaupilze im Obst-, Gemüse-, Getreide- und Zierpflanzenbau.

Die neuen Verbindungen der allgemeinen Formel I eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die neuen Verbindungen der Formel I können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Die nachfolgenden Beispiele dienen zur näheren Erläuterung der Erfindung, ohne dieselbe einzuschränken.

Beispiele für das Herstellungsverfahren Variante F)

Beispiel 1

Zu einer Lösung von 3,95 g (12 mMol) 2-S,(R)-(2,4-Dichlorphenyl)-2-(imidazol-1-ylmethyl)-4-R,(S)-hydroxymethyl-1,3-dioxolan (cis-Form) in 36 ml abs. N,N-Dimethylformamid (DMF) wurden 0,43 g (14,4 mMol) 80%ige Natriumhydrid-Öl-Dispersion zugesetzt. Man rührt 30 Min. bei ca. 45°C, wonach die Wasserstoffentwicklung abklang. Dann tropfte man in 5 Min. eine Lösung von 2,15 g (14 mMol) 2-Chlorbenzoxazol in 3 ml abs. DMF zu und rührte 17 Std. bei 70°C nach. Anschließend wurde das DMF unter Vakuum (3-8 mbar) abdestilliert. Den Rückstand versetzte man mit 50 ml Wasser und 50 ml $CH_2Cl_2$, schüttelte durch, trennte die Phasen und extrahierte die wäßrige Phase noch dreimal mit $CH_2Cl_2$. Die vereinigten $CH_2Cl_2$-Extrakte wurden mit $Na_2SO_4$ getrocknet, filtriert und im Vakuum eingedampft. Der Rückstand, 6,4 g zähes Öl, wurde an einer Kieselgel/$CH_2Cl_2$-Säule (⌀ 2,6 cm, Höhe 30 cm) chromatographiert. Verwendet wurde Kieselgel S, Riedel-de Haen, Korngröße 0,063-0,2 mm. Man eluierte fraktionsweise mit $CH_2Cl_2$ und überprüfte die Fraktionen dünnschichtchromatographisch (DC) (DC-Fertigplatten, Kieselgel 60, F 254, Merck). Auf diese Weise wurden 4,6 g dünnschichtchromatographisch nahezu reines 2-S,(R)-(2,4-Dichlorphenyl)-2-(imidazol-1-ylmethyl)-4-R,(S)-(benzoxazol-2-yloxymethyl)-1,3-dioxolan als zähes Öl erhalten (Ausbeute ≙ 85%).

Elementaranalyse: $C_{21}H_{17}Cl_2N_3O_4$ (MG 446,31) Ber. C 56,52, H 3,48, N 9,42, Gef. C 56,1, H 3,8, N 9,1%; das $^1$H-NMR-Spektrum ($CDCl_3$) bestätigt die erwartete Struktur.

Nitrat-Bildung: 2,23 g (5 mMol) der vorstehend erhaltenen, zähöligen Verbindung wurden in einer Mischung von 10 ml Ethylacetat und 15 ml Ether gelöst und unter Rühren mit 5 ml einer 1 m $HNO_3$/Ethylacetat-Lösung versetzt. Dabei fiel kristalliner Niederschlag (Nitrat) aus.

Man tropfte Ether zu, bis keine weitere Trübung der Lösung eintrat, saugte das Kristallisat ab, wusch mit Ether nach und trocknete das kristalline Produkt. Man erhielt 2,04 g 2-S,(R)-(2,4-Dichlorphenyl)-2-(imidazol-1-ylmethyl)-4-R,(S)-(benzoxazol-2-yloxymethyl)-1,3-dioxolan-nitrat, Fp. 141-142°C;
Elementaranalyse: $C_{21}H_{18}Cl_2N_4O_7$ (MG 509,32) Ber. C 49,52, H 3,56, Cl 13,92, N 11,00, Gef. C 48,7, H 3,7, Cl 13,7, N 10,7%.

Bei im Prinzip gleicher Arbeitsweise wie vorstehend beschrieben können auch Dimethylsulfoxid, N,N-Dimethylacetamid oder N-Methylpyrrolidon-(2), sowie Gemische dieser Lösungsmittel mit z. B. Tetrahydrofuran, Dioxan, 1,2-Dimethoxyethan oder Acetonitril verwendet werden.

Beispiel 2

Gemäß Beispiel 1, jeweils unter Verwendung der entsprechenden Verbindung der Formel IIa (A:CH oder N, Ar:2,4-Dichlorphenyl; M:H) und der entsprechenden Verbindungen der Formel XIa wurden die folgenden in der Tabelle 1 aufgeführten Verbindungen der Formel I (L, m und p stets gleichzeitig Null, Z=O) hergestellt. Die jeweils unterschiedlichen Reste bzw. Indizes A, X, $R^2$ und n sind aus der Tabelle 1 ersichtlich.

TABELLE 1

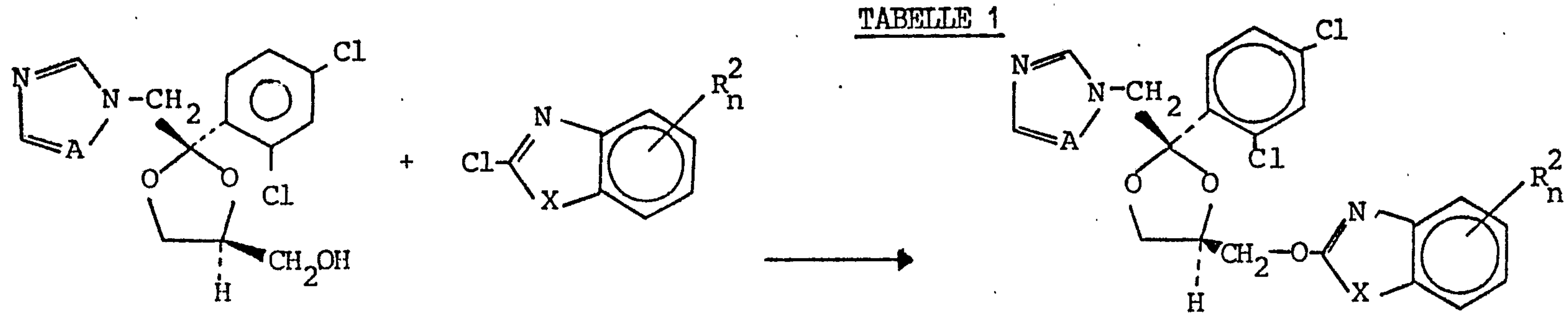

IIa (M=H) XIa I (L, m, p = 0, z = 0)

| Verb. Nr. | A | X | $R^2$ | n | Base | Fp.[°C] | Analyse Ber. | % Gef. | Salz | Fp.[°C] | Analyse Ber. | % Gef. | 2,4 Isomere *) |
|---|---|---|---|---|---|---|---|---|---|---|---|---|---|
| 1.1 | CH | O | 6-Cl | 1 | Base | 148-50 | C 52,47<br>H 3,36<br>N 8,74 | 52,7<br>3,4<br>8,8 | Nitrat | 162-63 | C 46,39<br>H 3,15<br>N 10,30 | 46,4<br>3,4<br>10,1 | cis |
| 1.2 | N | O | – | 0 | Base | 126-27 | C 53,71<br>H 3,61<br>N 12,53 | 53,3<br>3,3<br>12,7 | – | | | | cis |
| 1.3 | CH | O | 5,6-$(CH_3)_2$- | 2 | Base | 136-37 | C 58,24<br>H 4,46<br>N 8,86 | 57,9<br>4,3<br>8,6 | – | | | | cis |
| 1.4 | CH | S | – | 0 | Base | | C 54,55<br>H 3,71<br>N 9,09 | 54,2<br>3,8<br>9,2 | Nitrat | 127-28 | C 48,01<br>H 3,45<br>N 10,66 | 48,4<br>3,7<br>10,6 | cis |
| 1.5 | CH | S | 6-Cl | 1 | Base | | C 50,77<br>H 3,25<br>N 8,46 | 49,9<br>3,4<br>8,4 | Nitrat | 189-90 | C 45,05<br>H 3,06<br>N 10,01 | 45,1<br>3,3<br>9,7 | cis |

TABELLE 1, Forts.

| Verb. Nr. | A | X | $R^2$ | n | Base | Fp.[°C] | Analyse Ber. | % Gef. | Salz | Fp.[°C] | Analyse Ber. | % Gef. | 2,4 Isomere *) |
|---|---|---|---|---|---|---|---|---|---|---|---|---|---|
| 1.6 | N | S | 6-Cl | 1 | Base | 131-32 | C 48,26<br>H 3,04<br>N 11,26 | 48,4<br>3,3<br>11,1 | - | | | | cis |
| 1.7 | CH | S | $6-OC_2H_5$ | 1 | Base | | C 54,54<br>H 4,18<br>N 8,30 | 53,8<br>4,3<br>8,3 | Nitrat | 189-90 | C 48,51<br>H 3,89<br>N 9,84 | 48,6<br>4,1<br>9,3 | cis |
| 1.8 | CH | $N-CH_3$ | - | 0 | Base | | C 57,53<br>H 4,39<br>N 12,20 | 56,7<br>4,4<br>12,0 | - | | | | cis |

*) Cis- und Trans- bezieht sich auf den Azolylmethyl-Rest und den (subst.)-Oxymethyl-Rest in der 2-bzw. der 4-Stellung des Dioxolanringes

Beispiele für das Herstellungsverfahren - Variante A)

Beispiel 3

Zu einer Lösung von 1,84 g (11 mMol) 2-Mercaptobenzothiazol in 25 ml abs. DMF gab man 0,330 g (11 mMol) 80%ige Natriumhydrid-Öl-Dispersion. Nach Abklingen der Wasserstoffentwicklung wurde eine Lösung von 4,07 g (10 mMol) 2-S,(R)-(2,4-Dichlorphenyl)-2-(imidazol-1-ylmethyl)-4-R,(S)-methansulfonyloxymethyl-1,3-dioxolan in 13 ml abs. DMF hinzugegeben und 8 Stunden bei 90°C nachgerührt. Danach wurde das DMF am Rotationsverdampfer im Vakuum abgezogen und der Rückstand mit 30 ml Wasser und 40 ml $CH_2Cl_2$ versetzt und durchgeschüttelt. Nach Phasentrennung wurde die wäßrige Phase noch zweimal mit $CH_2Cl_2$ extrahiert. Die vereinigten $CH_2Cl_2$-Extrakte wurden getrocknet, filtriert und im Vakuum eingedampft. Der Rückstand wurde an einer Kieselgel(Kieselgel S, Riedel-de Haen, 0,063-0,2 mm)-$CH_2Cl_2$-Säule analog wie in Beispiel 1 beschrieben chromatographiert. Die laut DC einheitlichen Fraktionen wurden vereinigt und im Vakuum von Lösungsmittel befreit. Man erhielt 4,03 g (84% d.Th.) 2-S,(R)-(2,4-Dichlorphenyl)-2-(imidazol-1-ylmethyl)-4-R,(S)-(benzothiazol-2-ylthiomethyl)-1,3-dioxolan als hochviskoses Öl,

Analyse: $C_{21}H_{17}Cl_2N_3O_2S_2$ (MG 478.44), Ber. C 52,72, H 3,58, N 8,78, S 13,40; Gef. C 52,1, H 3,5, N 8,6, S 13,1.

Beispiel 4

Eine Lösung von 4,07 g (10 mMol) Cis-2-(2,4-Dichlorphenyl)-2-(imidazol-1-ylmethyl)-4-methansulfonyloxymethyl-1,3-dioxolan, 2,0 g (13 mMol) 2-Mercaptobenzothiazol und 1,5 g (15 mMol) Triethylamin in 25 ml Acetonitril wurde 16 h unter Rückfluß gekocht. Anschließend wurde das Lösungsmittel am Rotationsverdampfer im Vakuum abgezogen und der verbleibende Rückstand wie im Beispiel 3 beschrieben aufgearbeitet und an Kieselgel S chromatographiert. Man erhielt 4,3 g (90% d. Th. ) 2-S(R)-(2,4-Dichlorphenyl)-2-(imidazol-1-ylmethyl)-4-R,(S)-benzothiazol-2-ylthiomethyl)-1,3-dioxolan als zähes Öl ($^1$H-NMR-Spektrum und DC-Verhalten sind identisch mit der gemäß Beispiel 3 erhaltenen Substanz).

Beispiel 5

Gemäß Beispiel 3, jeweils unter Verwendung der Methansulfonate IIb bzw. IIc (vgl. Tabelle 2) und der entsprechenden Verbindungen der Formel IIIb (M=H, Z=S, L, m und p = 0) wurden die folgenden, in der Tabelle 2 aufgeführten Verbindungen der Formel I (Z=S, L, m und p=0) hergestellt.

TABELLE 2

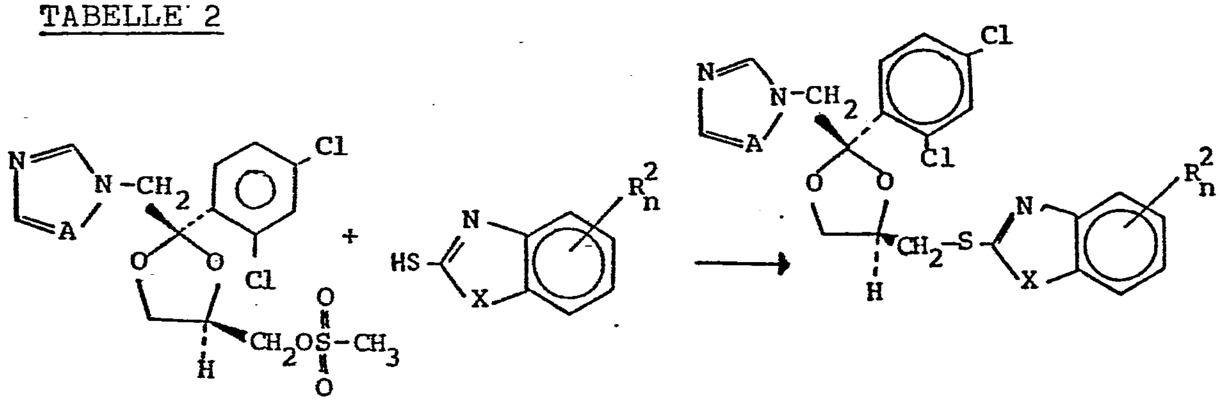

IIb (A=CH) IIIb I (L, m, p=0, Z=S)
IIc (A=N)

| Verb. Nr. | A | X | $R^2$ | n | Analyse % Ber. | Gef. | 2,4-Isomere |
|---|---|---|---|---|---|---|---|
| 2.1 | CH | O | – | 0 | C 54,55 | 53,9 | cis |
| | | | | | H 3,71 | 3,7 | |
| | | | | | N 9,09 | 8,9 | |
| | | | | | S 6,94 | 7,2 | |
| 2.2 | N | O | – | 0 | C 51,84 | 51,9 | cis |
| | | | | | H 3,48 | 3,4 | |
| | | | | | N 12,09 | 12,0 | |
| | | | | | S 6,92 | 7,0 | |

TABELLE 2, Forts.

| Verb. Nr. | A | X | $R^2$ | n | Analyse Ber. | % Gef. | 2,4-Isomere |
|---|---|---|---|---|---|---|---|
| 2.3 | CH | S | 7-CO-$C_6H_5$ | 1 | C 57,73 | 57,1 | cis |
| | | | | | H 3,63 | 3,8 | |
| | | | | | N 7,21 | 7,2 | |
| 2.4 | CH | S | 7-$COOCH_3$ | 1 | C 51,49 | 50,8 | cis |
| | | | | | H 3,57 | 3,65 | |
| | | | | | N 7,83 | 7,75 | |
| 2.5 | CH | S | 7-Cl | 1 | C 49,18 | 49,0 | cis |
| | | | | | H 3,14 | 3,2 | |
| | | | | | N 8,19 | 8,1 | |
| 2.6 | CH | S | 6-$CH_3$ | 1 | C 53,66 | 53,4 | cis |
| | | | | | H 3,89 | 3,9 | |
| | | | | | N 8,53 | 8,4 | |
| 2.7 | CH | S | 6-$OC_4H_9$ | 1 | C 54,54 | 53,6 | cis |
| | | | | | H 4,58 | 4,5 | |
| | | | | | N 7,63 | 7,3 | |
| | | | | | S 11,65 | 11,5 | |
| 2.8 | CH | N-$CH_3$ | - | 0 | C 55,58 | 55,2 | cis |
| | | | | | H 4,24 | 4,4 | |
| | | | | | N 11,79 | 11,5 | |

Beispiele für das Herstellungsverfahren - Variante E)

Beispiel 6

Zu einer Lösung von 3,29 g (10 mMol) Cis-2-(2,4-Dichlorphenyl)-2-(imidazol-1-ylmethyl)-4-hydroxymethyl-1,3-dioxolan in 40 ml abs. DMF wurden 0,34 g (11,3 mMol) 80%ige Natriumhydrid-Öl-Dispersion gegeben. Nach 25 Min. Rühren bei 40°C, wonach die $H_2$-Entwicklung abgeklungen war, wurde bei 40°C eine Lösung von 1,85 g (11 mMol) 2-Chlormethylbenzoxazol in 7 ml abs. DMF zugetropft und die Mischung 2,5 Std. bei 50°C nachgerührt. Nach Abdestillieren des DMF am Rotationsverdampfer im Vakuum wurde wie im Beispiel 1 beschrieben der verbliebene Rückstand weiter aufgearbeitet. Der Methylenchlorid-Extrakt-Rückstand wurde an einer Kieselgel S(0,063-0,2 mm, ∅ 2,6 cm, Höhe 36 cm)-$CH_2Cl_2$-Säule chromatographiert. Man eluierte fraktionsweise mit $CH_2Cl_2$ und $CH_2Cl_2/C_2H_5OH$-Mischungen. Die im DC einheitlichen Fraktionen wurden vereinigt und im Vakuum eingedampft. Dabei fielen 1,26 g (27,4% d. Th.) reines 2-S,(R)-(2,4-Dichlorphenyl)-2-(imidazol-1-ylmethyl)-4-R,(S)-[(benzoxazol-2-ylmethyl-oxy)-methyl]-1,3-dioxolan als hochviskoses Öl an;

Analyse: $C_{22}H_{19}Cl_2N_3O_4$ (MG 460,33), Ber. C 57,40, H 4,16, N 9,13; Gef. C 56,8, H 4,4, N 8,8%; das $^1H$-NMR-Spektrum ($CDCl_3$) bestätigte die Struktur.

Beispiel 7

Nach der gleichen Arbeitsweise wie im Beispiel 6 beschrieben wurde ausgehend von 2-Chlormethyl-benzothiazol, anstelle von 2-Chlormethyl-benzoxazol, Cis-2-(2,4-Dichlorphenyl)-2-(imidazol-1-ylmethyl)-4-[benzothiazol-2-ylmethyloxy)-methyl]-1,3-dioxolan als zähflüssiges Öl in 17%iger Ausbeute erhalten,

Analyse: $C_{22}H_{19}Cl_2N_3O_3S$ (MG 476,40) Ber. C 55,47 H 4,02, N 8,82; Gef. C 55,3 , H 4,3 , N 8,6 %.

Beispiel 8

Nach der gleichen Arbeitsweise wie im Beispiel 6 beschrieben wurde ausgehend von 2-Chlormethyl-6-chlor-benzothiazol und Cis-2-(4-Chlorphenyl)-2-(imidazol-1-ylmethyl)-4-hydroxymethyl-1,3-dioxolan Cis-2-(4-Chlorphenyl)-2-(imidazol-1-ylmethyl)-4-[(6-chlor-benzothiazol-2-ylmethyloxy)-methyl]-1,3-dioxolan als zähflüssiges Öl in 19%iger Ausbeute erhalten;

Analyse: $C_{22}H_{19}Cl_2N_3O_3S$ (MG 476,40) Ber. C 55,47, H 4,02, N 8,82, S 6,73; Gef. C 55,0, H 3,8, N 8,7, S 6,6%.

Beispiel 9

Zu einer Lösung von 4,9 g (15 mMol) Cis-2-(2,4-Dichlorphenyl)-2-(imidazol-1-ylmethyl)-4-hydroxymethyl-1,3-dioxolan in 50 ml abs. N,N-Dimethylformamid (DMF) wurden 0,55 g (18,2 mMol) 80%ige NaH-Öl-Dispersion gegeben. Man rührte 40 Min. bei 40°C, gab dann 5,25 g (18,2 mMol) 2-(4-Brommethylphenyl)-benzoxazol zu und rührte 16 Std. bei 80°C nach. Anschließend wurde wie im Beispiel 1 beschrieben aufgearbeitet. Der $CH_2Cl_2$-Extrakt-Rückstand (8,4 g zähes Öl) wurde an einer Kieselgel S-(ø 2,6 cm, Höhe 37 cm)-$CH_2Cl_2$-Säule wie im Beispiel 6 beschrieben chromatographiert. Als DC-einheitliche Fraktionen wurden, nach Abdunsten des Lösungsmittels im Vakuum, 4,38 g (54,5% d.Th.) kristallines, reines Cis-2-(2,4-Dichlorphenyl)-2-(imidazol-1-ylmethyl)-4-[(4-(benzoxazol-2-yl)-benzyl)-oxymethyl]-1,3-dioxolan erhalten. Die Substanz kann aus Methanol umkristallisiert werden (Fp. 111-112°C);

Analyse: $C_{28}H_{23}Cl_2N_3O_4$ (MG 536.43) Ber. C 62,69, H 4,32, N 7,83; Gef. C 62,4, H 4,4, N 7,6.

Nitrat-Bildung: 1,93 g (3,6 mMol) Base wurden in 15 ml Ethylacetat gelöst und mit 3,6 ml 1 m $HNO_3$/Ethylacetat unter Rühren versetzt. Man gab anschließend Ether zu, bis kein weiterer Niederschlag ausfiel und saugte ab. Man erhielt 2,05 g (95,3% d.Th. Ausbeute an Nitrat) Cis-2-(2,4-Dichlorphenyl)-2-(imidazol-1-ylmethyl)-4-[4-(benzoxazol-2-yl)-benzyl)oxymethyl]-1,3-dioxolan-nitrat; Fp. 121-2°C,

Analyse: $C_{28}H_{24}Cl_2N_4O_7$ (MG 599.45) Ber. C 56,10, H 4,04, H 9,35; Gef. C 55,5, H 4,0, N 9,2%.

Beispiel 10

Gemäß der in Beispiel 9 beschriebenen Arbeitsweise wurden hergestellt: Cis-2-(2,4-Dichlorphenyl)-2-(imidazol-1-yl-methyl)-4-[(4-(5-chlor-benzoxazol-2-yl)-benzyl)-oxymethyl]-1,3-dioxolan, Fp. 122-23°C, Analyse: $C_{28}H_{22}Cl_3N_3O_4$ (MG 570,88), Ber.: C 58,91, H 3,88, N 7,36; Gef. C 58,1 H 3,9, N 7,3%.

Cis-2-(2,4-Dichlorphenyl)-2-(imidazol-1-ylmethyl)-4-[(4-(benzothiazol-2-yl)-benzyl)-oxymethyl]-1,3-dioxolan, Analyse: $C_{28}H_{23}Cl_2N_3O_3S$ (MG 552,50), Ber. C 60,87, H 4,20, N 7,61; Gef. C 60,2, H 3,8, N 7,5%.

Cis-(4-Fluorphenyl)-2-(1,2,4-triazol-1-ylmethyl)-4-[(4-(benzothiazol-2-yl)-benzyl)-oxymethyl]-1,3-dioxolan, Fp. 108-9°C,

Analyse: $C_{27}H_{23}FN_4O_3S$ (MG 502,58), Ber. C 64,53, H 4,61, N 11,15, S 6,38; Gef. C 64,1, H 4,7, N 11,3, S 6,0%.

Weitere Beispiele für das Herstellungsverfahren - Variante A)

Beispiel 11

Zu einer Lösung von 2,11 g (10 mMol) 2-(4-Hydroxyphenyl)-benzoxazol in 50 ml abs. DMF gab man anteilweise bei 16-35°C 0,33 g (11 mMol) 80%ige NaH-Öl-Dispersion. Nach Abklingen der Wasserstoffentwicklung gab man 4,07 g (10 mMol) Cis-2-(2,4-Dichlorphenyl)-2-(imidazol-1-yl-methyl)-4-methansulfonyloxymethyl-1,3-dioxolan zu und rührte 7 Std. bei 75°C. Danach würden ca. 60% des DMF am Rotationsverdampfer im Vakuum abdestilliert. Der Rückstand wurde unter Rühren und Kühlen mit ca. 50 ml Wasser versetzt. Dabei fiel kristalliner Niederschlag aus. Nachdem noch 1 Std. bei 2-6°C gerührt worden war, saugte man das kristalline Produkt ab, trocknete es im Vakuum und kri-

stallisierte es aus Ethylacetat um. Dabei wurden 3,2 g (61,3% d. Th.) reines Cis-2-(2,4-Dichlorphenyl)-2-(imidazol-1-ylmethyl)-4-[(4-benzoxazol-2-yl)-phenoxy)-methyl]-1,3-dioxolan, Fp. 170-71°C, erhalten; Analyse: $C_{27}H_{21}Cl_2N_3O_4$ (MG 522,40), Ber. C 62,08, H 4,05, N 8,04; Gef. C 61,6, H 4,0, N 7,9%.

Nach der gleichen Arbeitsweise wurde ausgehend vom gleichen Methansulfonat und 5-Chlor-2-(4-hydroxyphenyl)-benzoxazol in 63%iger Ausbeute reines Cis-2-(2,4-Dichlorphenyl)-2-(imidazol-1-ylmethyl)-4-[(4-(5-chlor-benzoxazol-2-yl)-phenoxy)- methyl]-1,3-dioxolan, Fp. 196-7°C erhalten; Analyse: $C_{27}H_{20}Cl_3N_3O_4$ (MG 556,85), Ber. C 58,24, H 3,62, N 7,55; Gef. C 57,8, H 3,7 N 7,4%.

Beispiel 12

Zu einer Lösung von 1,48 g (5 mMol) 2-(4-(4-Hydroxyphenyl)-piperazin-1-yl)-benzoxazol (vgl. Beispiel 29 ) in 20 ml abs. N,N-Dimethylformamid (DMF) gab man bei Zimmertemperatur 154 mg (5,13 mMol) 80%ige Natriumhydrid-Öl-Dispersion. Nach Ende der Wasserstoffentwicklung wurde tropfenweise eine Lösung von 2-S,(R)-(2,4-Dichlorphenyl)-2-(imidazol-1-ylmethyl)-4(R),(S)-methansulfonyloxymethyl-1,3-dioxolan (cis-Form) in 15 ml abs. DMF zugegeben und 4,5 Std. bei 100°C nachgerührt. Diese Umsetzung wurde unter Überlagerung mit $N_2$-Gas ausgeführt. Anschließend wurde am Rotationsverdampfer im Vakuum (4-16 mbar) das DMF abdestilliert und der verbliebene Rückstand mit $CH_2Cl_2$ und Wasser versetzt und durchgeschüttelt. Die wäßrige Phase wurde mit verd. HCl auf pH ca. 6 gestellt. Nach Trennung der Phasen wurde die wäßrige noch zweimal mit $CH_2Cl_2$ extrahiert. Die vereinigten Methylenchloridextrakte wurden mit $Na_2SO_4$ getrocknet, filtriert und im Vakuum eingedampft. Der verbliebene kristalline Rückstand wurde aus Acetonitril umkristallisiert. Man erhielt 1,75 g (57,7 % d.Th.) reines 2-S,(R)-(2,4-Dichlorphenyl)-2-(imidazol-1-ylmethyl)-4-R,(S)-[4-(4-(benzoxazol-2-yl)-piperazin-1-yl)-phenoxymethyl]-1,3-dioxolan, Fp. 169-70; Analyse: $C_{31}H_{29}Cl_2N_5O_4$ (MG 606,53), Ber.

C 61,39, H 4,82, Cl 11,69, N 11,55; Gef. 60,7 H 4,8, Cl 12,0 N 11,3%.

Beispiel 13

Nach der gleichen Arbeitsweise wie im Beispiel 12 beschrieben unter Verwendung von 2-S,(R)-(2,4-Dichlorphenyl)-2-(imidazol-1-ylmethyl)- oder -(1,2,4-triazol-1-ylmethyl)-4-R,(S)-methansulfonyloxymethyl-1,3-dioxolan (IIb bzw. IIc) und jeweils des entsprechenden 4-(4-Hydroxyphenyl)-piperazin-1-yl-Derivats der Formel IIIc (M=H, Z=O, L=1 und m und p=O) wurden die in der Tabelle 3 aufgeführten Verbindungen der Formel Ia nach Verfahrensvariante A) hergestellt.

TABELLE 3

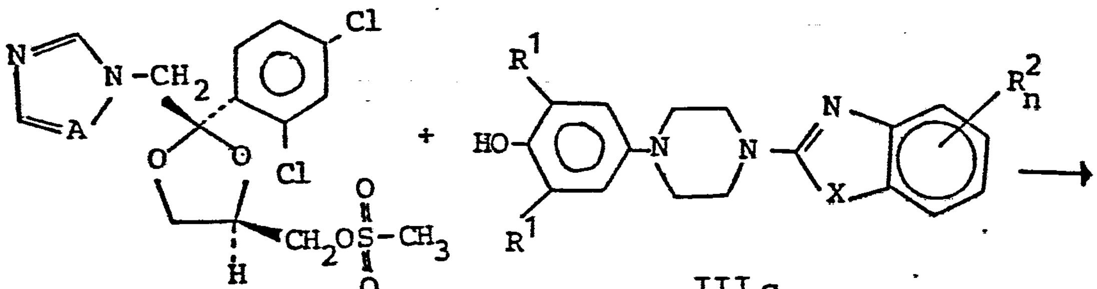

IIb (A=CH)
IIc (A=N)

IIIc

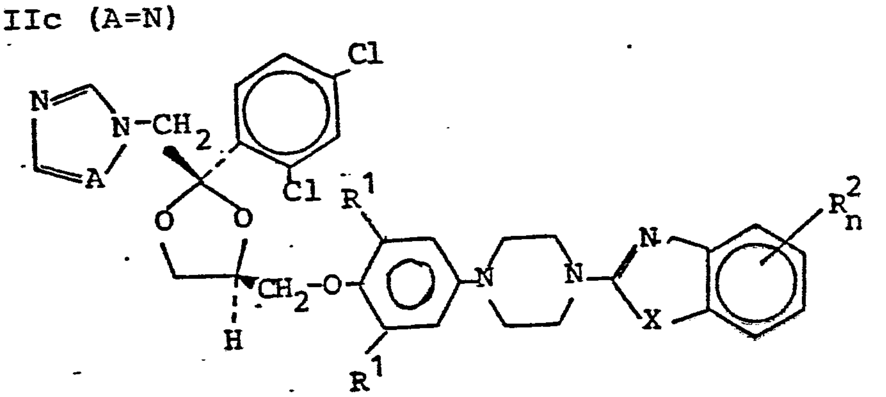

Ia (m, p=O, Z=O)

| Verb. Nr. | A | $R^1$ | X | $R^2$ | n | Analyse Ber. | % Gef. | Fp. [°C] |
|---|---|---|---|---|---|---|---|---|
| 3.1 | N | H | O | - | 0 | C 59,31 | 59,5 | 172-3 |
| | | | | | | H 4,65 | 4,6 | |
| | | | | | | N 13,83 | 13,7 | |

TABELLE 3, Forts.

| Verb-Nr. | A | $R^1$ | X | $R^2$ | n | Analyse Ber. | % Gef. | Fp. [°C] |
|---|---|---|---|---|---|---|---|---|
| 3.2 | CH | H | O | 6-Cl | 1 | C 58,09 | 57,9 | 144-5 |
| | | | | | | H 4,40 | 4,3 | |
| | | | | | | N 10,93 | 10,9 | |
| 3.3 | N | H | O | 6-Cl | 1 | C 56,13 | 55,9 | 139-40 |
| | | | | | | H 4,24 | 4,1 | |
| | | | | | | N 13,09 | 13,0 | |
| 3.4 | CH | H | O | 5-$CH_3$ | 1 | C 61,93 | 61,8 | 173-4 |
| | | | | | | H 4,90 | 5,0 | |
| | | | | | | N 11,29 | 11,0 | |
| 3.5 | CH | H | S | 6-Cl | 1 | C 56,67 | 56,2 | 121-2 |
| | | | | | | H 4,30 | 4,7 | |
| | | | | | | N 10,66 | 10,4 | |
| 3.6 | N | H | S | 6-Cl | 1 | C 54,76 | 54,0 | 146-7 |
| | | | | | | H 4,14 | 4,1 | |
| | | | | | | N 12,77 | 12,3 | |
| 3.7 | CH | H | S | 6-$OC_2H_5$ | 1 | C 59,46 | 59,4 | 172-3 |
| | | | | | | H 4,99 | 5,0 | |
| | | | | | | N 10,51 | 10,3 | |
| 3.8 | N | H | S | 6-$OC_2H_5$ | 1 | C 57,57 | 57,5 | 173-4 |
| | | | | | | H 4,83 | 4,8 | |
| | | | | | | N 12,59 | 12,2 | |
| 3.9 | CH | $CH_3$ | O | 5-$CH_3$ | 1 | C 62,96 | 62,6 | 160-1 |
| | | | | | | H 5,44 | 5,4 | |
| | | | | | | N 10,80 | 10,6 | |

TABELLE 3, Forts.

| Verb. Nr. | A | $R^1$ | X | $R^2$ | n | Analyse Ber. | % Gef. | Fp. [°C] |
|---|---|---|---|---|---|---|---|---|
| 3.10 | N | $CH_3$ | O | 5-$CH_3$ | 1 | C 61,01 | 61,2 | - |
| | | | | | | H 5,28 | 5,3 | |
| | | | | | | N 12,94 | 12,6 | |
| 3.11 | CH | $CH_3$ | S | 5-Cl | 2 | C 58,41 | 58,6 | - |
| | | | | 6-$CH_3$ | | H 4,90 | 4,9 | |
| | | | | | | N 10,02 | 9,8 | |
| | | | | | | S 4,59 | 4,7 | |

Beispiel 14

Eine Lösung von 3,71 g (12 mMol) 1-(4-Hydroxyphenyl)-4-(benzoxazol-2-ylmethyl)-piperazin in 45 ml abs. DMF wurde mit 370 mg (12,31 mMol) 80%iger NaH-Öl-Dispersion und anschließend nach Ende der $H_2$-Entwicklung mit einer Lösung von 5,0 g Cis-2-(2,4-Dichlorphenyl)-2-(imidazol-1-ylmethyl)-4-methansulfonyloxymethyl-1,3-dioxolan (IIb) in 35 ml abs. DMF versetzt und 3 Std. bei 95°C gerührt. Die anschließende Aufarbeitung erfolgte gemäß der im Beispiel 12 beschriebenen Weise. Der Methylenchlorid-Extrakt-Rückstand (8,5 g) wurde an einer Kieselgel S-$CH_2Cl_2$-Säule (ø 2,6 cm, Höhe 32 cm) unter fraktionsweiser Elution mit $CH_2Cl_2$ und $CH_2Cl_2$/EtOH-Mischungen chromatographiert. DC einheitliche Fraktionen wurden vereinigt und ergaben 5,7 g fast reines Cis-2-(2,4-Dichlorphenyl)-2-(imidazol-1-ylmethyl)-4-[4-(4-(benzoxazol-2-ylmethyl)-piperazin-1-yl)-phenoxymethyl]-1,3-dioxolan, das aus Methanol umkristallisiert wurde. Man erhielt 3,75 g reine Verbindung, Fp. 139-40°C, (50,3% d.Th.), Analyse: $C_{32}H_{31}Cl_2N_5O_4$ (MG 620,55) Ber. C 61,94, H 5,04, N 11,29; Gef. C 61,7, H 5,1, N 11,2%.

Beispiel 15

Nach im Prinzip gleicher Weise wie im Beispiel 14 beschrieben wurden 10 mMol 4-(4-Hydroxyphenyl)-1-(benzoxazol-2-yl-methyl)-piperazin in 90 ml abs. 1,2-Dimethoxyethan mit 1,19 g (10,6 mMol) Kalium-tert.-butylat und nach 10 Min. Rühren anschließend mit 10 mMol IIb versetzt und 6 Std. am Rückfluß gekocht. Nach Verdampfen des Lösungsmittels im Vakuum wurde wie im Beispiel 14 beschrieben aufgearbeitet und chromatographiert. Man erhielt 2,78 g (44,8% d. Th.) Cis-2-(2,4-Dichlorphenyl)-2-(imidazol-1-ylmethyl)-4-[4-(4-(benzoxazol-2-ylmethyl)-piperazin-1-yl)-phenoxymethyl]-1,3-dioxolan, Fp. 139-40°C nach Umkristallisation aus Methanol.

Beispiel 16

Man der gleichen Arbeitsweise wie im Beispiel 14 beschrieben, ausgehend von IIb oder IIc und jeweils dem entsprechenden 4-(4-Hydroxyphenyl)-piperazin-1-yl-Derivat der Formel IIId (M=H, Z=O, L=1) wurden die in der Tabelle 4 aufgeführten Verbindungen der Formel Ib nach Verfahrensvariante A) hergestellt.

TABELLE 4

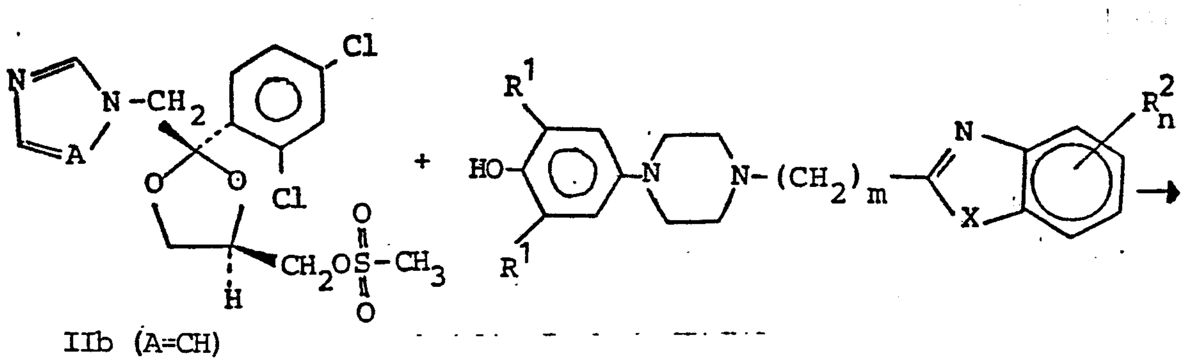

IIb (A=CH)

IIc (A=N)

IIId

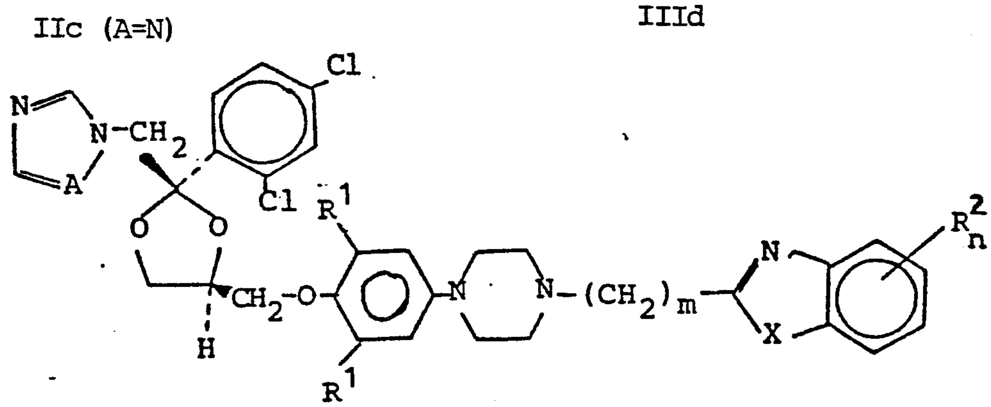

Ib (p=0, Z=0)

TABELLE 4, Forts.

| Verb. Nr. | A | $R^1$ | m | X | $R^2$ | n | Analyse Ber. | % Gef. | Fp. [°C] |
|---|---|---|---|---|---|---|---|---|---|
| 4.1 | N | H | 1 | O | – | 0 | C 59,91<br>H 4,87<br>N 13,52 | 60,0<br>5,0<br>13,4 | 148-9 |
| 4.2 | CH | H | 1 | O | $5.6\text{-}(CH_3)_2$ | 2 | C 62,96<br>H 5,44<br>N 10,80 | 63,1<br>5,6<br>10,4 | 156-7 |
| 4.3 | N | H | 1 | O | $5.6\text{-}(CH_3)_2$ | 2 | C 61,02<br>H 5,28<br>N 12,94 | 60,7<br>5,3<br>12,7 | 165-6 |
| 4.4 | CH | H | 1 | O | 5-Cl | 1 | C 58,68<br>H 4,62<br>N 10,69 | 58,7<br>4,9<br>10,6 | – |
| 4.5 | N | H | 1 | O | 5-Cl | 1 | C 56,76<br>H 4,46<br>N 12,80 | 56,6<br>4,4<br>12,4 | – |
| 4.6 | CH | H | 1 | S | – | 0 | C 60,37<br>H 4,91<br>N 11,00 | 59,6<br>4,9<br>10,7 | 135-6 |
| 4.7 | N | H | 1 | S | – | 0 | C 58,40<br>H 4,74<br>N 13,18 | 58,1<br>4,7<br>13,4 | 125-6 |
| 4.8 | CH | H | 1 | O | 4,5- 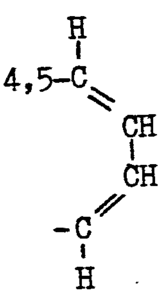 | 2 | C 64,48<br>H 4,96<br>N 10,45 | 64,2<br>4,9<br>10,0 | 183-4 |

TABELLE 4, Forts.

| Verb. Nr. | A | $R^1$ | m | X | $R^2$ | n | Analyse Ber. | % Gef. | Fp. [°C] |
|---|---|---|---|---|---|---|---|---|---|
| 4.9 | CH | $CH_3$ | 1 | O | - | 0 | C 62,96<br>H 5,44<br>N 10,80 | 62,7<br>5,3<br>10,8 | - |
| 4.10 | CH | $CH_3$ | 1 | S | - | 0 | C 61,44<br>H 5,31<br>N 10,54 | 61,1<br>5,0<br>10,3 | - |
| 4.11 | CH | H | 3 | O | - | 0 | C 62,96<br>H 5,44<br>N 10,80 | 62,4<br>5,5<br>10,5 | - |
| 4.12 | N | H | 3 | O | - | 0 | C 61,02<br>H 5,28<br>N 12,94 | 60,8<br>5,2<br>12,5 | 83-4 |
| 4.13 | CH | H | 3 | O | 5-Cl | 1 | C 59,79<br>H 5,02<br>N 10,25 | 58,9<br>5,0<br>9,9 | - |
| 4.14 | CH | H | 3 | S | - | 0 | C 61,44<br>H 5,31<br>N 10,54 | 61,3<br>5,4<br>10,4 | - |
| 4.15 | CH | $CH_3$ | 3 | O | - | 0 | C 63,90<br>H 5,81<br>N 10,35 | 62,9<br>5,7<br>9,8 | - |

TABELLE 4, Forts.

| Verb. Nr. | A | $R^1$ | m | X | $R^2$ | n | Analyse Ber. | % Gef. | Fp. [°C] |
|---|---|---|---|---|---|---|---|---|---|
| 4.16 | N | $CH_3$ | 3 | 0 | - | 0 | C 62,04 | 61,8 | - |
| | | | | | | | H 5,65 | 5,7 | |
| | | | | | | | N 12,40 | 12,2 | |
| 4.17 | CH | $CH_3$ | 3 | S | - | 0 | C 62,42 | 62,0 | - |
| | | | | | | | H 5,68 | 5,4 | |
| | | | | | | | N 10,11 | 10,1 | |
| | | | | | | | S 4,62 | 4,3 | |

Beispiel 17

Eine Lösung von 1,93 g (5 mMol) 1-(4-Hydroxyphenyl)-4-(4-(benzoxazol-2-yl)-benzyl)-piperazin in 20 ml abs. DMF wurde mit 154 mg (5.13 mMol) 80%iger NaH-Öl-Dispersion und nach Ende der $H_2$-Entwicklung mit einer Lösung von 2,09 g (5,14 mMol) Cis-2-(2,4-Dichlorphenyl)-2-(imidazol-1-ylmethyl)-4-methansulfonyloxymethyl-1,3-dioxolan (IIb) in 15 ml abs. DMF versetzt und 8 Std. bei 100°C gerührt. Die anschließende Aufarbeitung erfolgte gemäß der im Beispiel 12 beschriebenen Arbeitsweise. Der Methylenchlorid-Extrakt-Rückstand (3,5 g) wurde an einer Kieselgel S-$CH_2Cl_2$-Säule (⌀ 2,0 cm, Höhe 30 cm) wie im Beispiel 14 beschrieben chromatographiert. Man erhielt nach Vereinigung und Eindampfen der einheitlichen Fraktionen 1,79 g (51% d.Th.) Cis-(2,4-Dichlorphenyl)-2-(imidazol-1-ylmethyl)-4-[4-(4-(4-(benzoxazol-2-yl)-benzyl)-piperazin-1-yl)-phenoxymethyl]-1,3-dioxolan als hochviskoses Öl;

Analyse: (MG 696,65) Ber. C 65,52, H 5,06, N 10,05; Gef. C 65,6, H 5,1, N 9,7%.

Mit der gleichen Arbeitsweise wurde ausgehend vom Methansulfonat IIc die entsprechende 1,2,4-Triazol-1-ylmethyl-Verbindung, Cis-2-(2,4-Dichlorphenyl)-2-(1,2,4-triazol-1-ylmethyl)-4-[4-(4-(4-(benzoxazol-2-yl)-benzyl)-piperazin-1-yl)-phenoxymethyl]-1,3-dioxolan als hochwirksames Öl erhalten. Diese Substanz wurde in Methanol mit HCl/Ether in das Monohydrochlorid, Fp. 140-1°C, übergeführt, Analyse: $C_{37}H_{35}Cl_3N_6O_4$ (MG 734,11), Ber. C 60,54, H 4,81, N 11,45, $Cl^{\ominus}$ 4,83; Gef. C 60,0, H 4,8, N 10,9, $Cl^{\ominus}$ 5,3%; Ausbeute: 32% d. Th.

Beispiel 18

Gemäß der Arbeitsweise wie in Beispiel 17 beschrieben wurden hergestellt: Cis-2-(2,4-Dichlorphenyl)-2-(imidazol-1-ylmethyl)-4-[4-(4-(4-(benzothiazol-2-yl)-benzyl)-piperazin-1-yl)-phenoxymethyl]-1,3-dioxolan, Fp. 156-7°C, Analyse: $C_{38}H_{35}Cl_2N_5O_3S$ (MG 712,72) Ber. C 64,04, H 4,95, N 9,83; Gef. C 64,3, H 5,0, N 9,8%,
Cis-2-(2,4-Dichlorphenyl)-2-(1,2,4-triazol-1-ylmethyl)-4-[4-(4-(4-(benzothiazol-2-yl)-benzyl-piperazin-1-yl)-phenoxymethyl]-1,3-dioxolan, Fp. 164-5°, Analyse: $C_{37}H_{34}Cl_2N_6O_3S$ (MG 713,71) Ber. C 62,27, H 4,80, N 11,78; Gef. C 62,5, H 4,8, N 11,7%.

Beispiel 19

Zu einer frisch hergestellten Lösung von 230 mg Natrium in 20 ml abs. Ethanol tropfte man bei Raumtemperatur unter Rühren eine Lösung von 3,25 g (10 mMol) 4-(4-Hydroxyphenyl)-1-(benzothiazol-2-ylmethyl)-piperazin in 30 ml abs. Ethanol, rührte 15 Min. nach, gab 4,07 g IIb portionsweise bei Raumtemperatur zu und kochte anschließend 6 Std. unter Rückfluß. Danach wurde das Ethanol im Vakuum am Rotationsverdampfer abdestilliert und der Rückstand in 80 ml $CH_2Cl_2$ und 25 ml Wasser aufgenommen. Nach Durchmischung und Phasentrennung wurde die wäßrige Phase noch zweimal mit $CH_2Cl_2$ extrahiert. Die vereinigten $CH_2Cl_2$-Extrakte wurden getrocknet, filtriert und im Vakuum eingedampft. Der Extrakt-Rückstand (6,7 g) wurde in 15 ml Ethylacetat gelöst und mit Ether bis kurz vor die Trübungsgrenze versetzt.

Nach Anreiben erfolgte Kristallisation. Das abgesaugte Produkt wurde nochmals aus Ethylacetat umkristallisiert. Man erhielt 3,92 g reines Cis-2-(2,4-Dichlorphenyl)-2-(imidazol-1-ylmethyl)-4-[4-(4-(benzothiazol-2-ylmethyl)-piperazin-1-yl)-phenoxymethyl]-1,3-dioxolan, Fp. 135-36°C, (vgl. Beispiel 16, Tabelle 4, Nr. 4.6).

Beispiel 20

Eine Mischung aus 2,56 g (7,6 mMol) 4-(4-Hydroxy-3,5-dimethyl-phenyl)-1-(benzoxazol-2-ylmethyl)-piperazin, 40 ml Toluol, 8 ml 50%ige Natronlauge, 2,86 g (7 mMol) IIb (vgl. Tab. 2) und 0,35 Tetrabutylammoniumbromid wurde 3,5 Std. bei 100°C intensiv gerührt. Danach wurden die Phasen getrennt und die konz. Natronlauge zweimal mit Ether ausgewaschen. Die vereinigten organischen Phasen wurden zweimal mit Wasser ausgewaschen, getrocknet, filtriert und im Vakuum eingedampft. Der Extraktrückstand (4,3 g) wurde an einer Kieselgel S-$CH_2Cl_2$-Säule (ø 2,0 cm, Höhe 30 cm) wie im Beispiel 14 beschrieben chromatographiert. Man erhielt durch Vereinigen und Eindampfen DC einheitlicher Fraktionen 3,15 g (69,4% d.Th.) reines Cis-2-(2,4-Dichlorphenyl)-2-(imidazol-1-ylmethyl)-4-[4-(4-(benzoxazol-2-ylmethyl)-piperazin-1-yl)-2,6-dimethyl-phenoxymethyl]-1,3-dioxolan (vgl. auch Beispiel 16, Tabelle 4, Nr. 4.8),
Analyse: Gef. C 62,6, H 5,3, N 10,5%.

Nach der gleichen Arbeitsweise wurde unter Anwendung von IIb und der entsprechenden Benzothiazol-2-ylmethyl-piperazin-Verbindung Cis-2-(2,4-Dichlorphenyl)-2-(imidazol-1-ylmethyl)-4-[4-(4-(benzothiazol-2-ylmethyl)-piperazin-1-yl)-2,6-dimethyl-phenoxymethyl]-1,3-dioxolan in 79,3%iger Ausbeute erhalten (vgl. auch Beispiel 16, Tabelle 4, Nr. 4.9); Analyse: Gef. C 61,2, H 5,1, N 10,2%, und unter Anwendung von IIb bzw. IIc und 4-(4-Hydroxy-3,5-dimethyl-phenyl)-1-(5-methyl-benzoxazol-2-yl)-piperazin wurden Cis-2-(2,4-Dichlorphenyl)-2-(imidazol-1-ylmethyl)-4-[4-(4-(5-methyl-benzoxazol-2-yl)-piperazin-1-yl)-2,6-dimethyl-phen-

oxymethyl]-1,3-dioxolan, Ausbeute 81,2%, Fp. 160°C, Analyse: Ber. C 62,96, H 5,4, N 10,80; Gef. C 62,5, H 5,3, N 10,6%; und die entsprechende Triazolverbindung Cis-2-(2,4-Dichlorphenyl)-2-(1,2,4-triazol-1-ylmethyl)-4-[4-(4-(5-methylbenzoxazol-2-yl)-piperazin-1-yl)-2,6-dimethyl-phenoxymethyl]-1,3-dioxolan, Ausbeute 78,4%, hochviskoses Öl, erhalten.
Analyse: Ber. C 61,01, H 5,28, N 12,94; Gef. C 61,1, H 5,1, N 12,7% erhalten.

Weiterhin wurde nach der gleichen Arbeitsweise ausgehend von der IIb analogen 2-(4-Chlorphenyl)-Verbindung und dem entsprechenden Piperazin-Derivat Cis-2-(4-Chlorphenyl)-2-(imidazol-1-ylmethyl)-4-[4-(4-(5-ethyl- benzoxazol-2-yl)-piperazin-1-yl)-phenoxymethyl]-1,3-dioxolan, Fp.106-8°C in 70,2%iger Ausbeute erhalten,
Analyse: $C_{33}H_{34}ClN_5O_4$ (MG 600,13) Ber. C 66,05, H 5,71, N 11,67; Gef. C 65,7, H 5,6 N 11,3%.

Beispiele für das Herstellungsverfahren Variante B)

Beispiel 21

a) Zu einer Lösung von 6,19 g (20 mMol) 1-(4-Hydroxyphenyl)-4-(benzoxazol-2-ylmethyl)-piperazin in 70 ml abs. N,N-Dimethylformamid (DMF) gab man bei Raumtemperatur (unter Kühlung) 0,60 g 80%ige Natriumhydrid-Öl-Dispersion. Nach Ende der Wasserstoffentwicklung wurde bei Raumtemperatur eine Lösung von 8,40 g (20 mMol) Cis-2-Brommethyl-2-(2,4-dichlorphenyl)-4-methansulfonyloxymethyl-1,3-dioxolan (cis und trans bezieht sich auf die Brommethyl- und Methansulfonyloxymethyl-Gruppe in der 2- bzw. 4-Stellung des Dioxolanrings) in 25 ml abs. DMF zugetropft und die Mischung 8 Std. bei 60°C gerührt. Die anschließende Aufarbeitung und Chromatographie erfolgte in der gleichen Weise wie im Beispiel 14 beschrieben. Die chromatographische Reinigung erfolgte an einer Kieselgel S-Methylenchlorid/Petrolether 2:1-Säule (ø 3,6 cm, Höhe 44 cm). Man erhielt 8,6 g (68% d. Th.) Cis-2-Brommethyl-2-(2,4-dichlorphenyl)-4-[4-(4-benzoxazol-2-ylmethyl)-piperazin-1-yl)-phenoxymethyl]-1,3-dioxolan als zähes Öl.

| Analyse: | Ber. | | Gef. | |
|---|---|---|---|---|
| $C_{29}H_{28}BrCl_2N_3O_4$ | C | 54,99 | C | 54,1 |
| (MG 633,40) | H | 4 46 | H | 4,2 |
| | Br | 12,62 | Br | 12,5 |
| | Cl | 11,20 | Cl | 11,3 |
| | N | 6,63 | N | 6,3 |

b) Zu einer Lösung von 0,782 g (11,5 mMol) Imidazol in 25 ml abs. N,N-Dimethylacetamid gab man 0,345 g (11,5 mMol) 80%ige NaH-Öl-Dispersion. Nach Ende der Wasserstoffentwicklung wurde eine Lösung von 6,33 g (10 mMol) der unter a) hergestellten Brommethylverbindung in 10 ml abs. N,N-Dimethylacetamid zugetropft und anschließend 24 Std. am Rückfluß gekocht. Danach wurde wie im Beispiel 14 beschrieben aufgearbeitet und chromatographiert. Das Dimethylacetamid wurde im Ölpumpenvakuum am Rotationsverdampfer abdestilliert. Nach Chromatographie wurden 2,42 g noch schwach verunreinigtes Cis-2-(2,4-Dichlorphenyl)-2-(imidazol-1-ylmethyl)-4-[4-(4-(benzoxazol-2-ylmethyl)-piperazin-1-yl)-phenoxymethyl]-1,3-dioxolan erhalten, die aus Methanol umkristallisiert 2,07 g (33,4% d. Th.) reines Produkt (vgl. Beispiel 14), Fp. 139-40°C ergaben.

Beispiel 22

a) Nach der gleichen Arbeitsweise wie im Beispiel 21a beschrieben wurde ausgehend von 1-(4-Hydroxyphenyl)-4-(6-chlor-benzothiazol-2-yl)-piperazin und Cis-2-Brommethyl-2-(2,4-dichlorphenyl)-4-methansulfonyloxymethyl-1,3-dioxolan in 65%iger Ausbeute Cis-2-Brommethyl-2-(2,4-dichlorphenyl)-4-[4-(4-(6-Chlor-benzothiazol-2-yl)-piperazin-1-yl)-phenoxymethyl]-1,3-dioxolan, Fp. 129-130°C, hergestellt;

| Analyse: | Ber. | | Gef. | |
|---|---|---|---|---|
| $C_{28}H_{25}BrCl_3N_3O_3S$ | C | 50,20 | C | 50,3 |
| (MG 669,89) | H | 3,76 | H | 3,7 |
| | Br | 11,93 | Br | 12,1 |
| | Cl | 15,88 | Cl | 16,0 |
| | N | 6,27 | N | 6,1 |
| | S | 4,79 | S | 5,2 |

b) Nach der gleichen Arbeitsweise wie im Beispiel 21b beschrieben wurde ausgehend von 0,794 g (11,5 mMol) 1,2,4-Triazol, 0,345 g (11,5 mMol) 80%iger NaH-Öl-Dispersion und 6,7 g (10 mMol) von dem unter a) hergestellten Cis-2-Brommethyl-2-(2,4-dichlorphenyl)-4-[4-(4-(6-chlor-benzothiazol-2-yl)-piperazin-1-yl)-phenoxymethyl]-1,3-dioxolan in abs. N,N-Dimethylacetamid Cis-2-(2,4-Dichlorphenyl)-2-(1,2,4-triazol-1-ylmethyl)-4-[4-(4-(6-chlorbenzothiazol-2-yl)-piperazin-1-yl)-phenoxymethyl]-1,3-dioxolan, (2,05 g ≙ 31,2% Ausbeute, Fp. 146-47°C, hergestellt. Die Substanz war nach der chromatographischen Reinigung aus Methanol umkristallisiert worden.

Beispiel 23

Zu einer Suspension von 0,49 g (16,3 mMol) 80%iger NaH-Öl-Dispersion in 15 ml abs. Dimethylsulfoxid (DMSO) tropfte man bei Zimmertemperatur eine Lösung von 1,023 g (14,83 mMol) 1,2,4-Triazol in 4 ml abs. DMSO, rührte 30 Min. bei Zimmertemperatur nach und gab anschließend 6,7 g (10 mMol) gemäß Beispiel 22a hergestelltes Cis-2-Brommethyl-2-(2,4-dichlorphenyl)-4-[4-(4-(6-chlor-benzothiazol-2-yl)-piperazin-1-yl)-phenoxymethyl]-1,3-dioxolan, gelöst in 6 ml abs. DMSO, tropfenweise zu und rührte 16 Std. bei 130°C unter Stickstoffüberlagerung. Nach Abkühlung wurde die Reaktionsmischung in 100 ml Wasser eingerührt. Diese Mischung extrahierte man mehrmals mit $CH_2Cl_2$. Die vereinigten $CH_2Cl_2$-Extrakte wurden getrocknet, filtriert und im Vakuum eingedampft. Der Rückstand (6,7 g) wurde wie im Beispiel 14 erwähnt durch Säulenchromatographie an Kieselgel S gereinigt. Die vereinigten DC einheitlichen Fraktionen lieferten nach dem Eindampfen einen kristallinen Rückstand, der mit Hexan angemischt und abgesaugt wurde. Man erhielt 2,62 g (39,8% d. Th.) reine Verbindung, Fp. 146-7°C (vgl. auch Beispiel 13, Tabelle 3, Nr. 3.5).

Beispiel 24

Eine Mischung von 2,42 g (7 mMol) 1-(4-Hydroxyphenyl)-4-(6-chlorbenzothiazol-2-yl)-piperazin, 100 ml Toluol, 2,94 g (7 mMol) 2-Brommethyl-2-(2,4-dichlorphenyl)-4-methansulfonyloxymethyl-1,3-dioxolan, 0,50 g Tetrabutylammoniumbromid und 14 ml 50%ige Natronlauge wurden 12 Std. bei 65°C intensiv gerührt. Anschließend wurde die abgekühlte Mischung abgesaugt, um als Feststoff verbliebenes Hydroxyphenyl-(6-chlorbenzothiazol-2-yl)-piperazin abzutrennen. Von diesem Ausgangsstoff wurden auf diese Weise 0,58 g (≙ 24% des Einsatzes) unverändert zurückerhalten. Die Phasen des klaren Filtrats wurden getrennt. Die Toluollösung wurde zweimal mit Wasser ausgeschüttelt, getrocknet, filtriert und im Vakuum eingedampft. Der verbliebene Rückstand wurde kochend in Acetonitril gelöst. Beim Kühlen der Lösung fiel kristalliner Niederschlag aus. Dieser wurde weitere zweimal aus Acetonitril (zwischendurch heiß filtriert) umkristallisiert. Man erhielt 2,49 g (70% d. Th. bezogen auf umgesetztes Phenolderivat) reines 2-Brommethyl-2-(2,4-dichlorphenyl)-4-[4-(4-(6-chlor-benzothiazol-2-yl)-piperazin-1-yl)-phenoxymethyl]-1,3-dioxolan (Cis-trans-Diasteromeren-Mischung), Fp. 115-16°C, Analyse: Gef. C 50,2 , H 3,7, Br 12,2, Cl 15,9, N 6,3, S 5,3 (Ber. vgl. Beispiel 22a)

Beispiele für das Herstellungsverfahren Variante C)

Beispiel 25

Eine Mischung von 300 ml Benzol und 10,5 g (55 mMol) p-Toluolsulfonsäure-monohydrat wurde zunächst am Wasserabscheider wasserfrei gekocht. In die abgekühlte Lösung gab man anschließend 17,1 g (40 mMol) α-(Imidazol-1-yl)-2,4-dichlor-acetophenon-p-toluolsulfonat, 150 ml n-Butanol und 11,58 g (48 mMol) 1-(Benzothiazol-2-ylthio)-2,3-dihydroxypropan und kochte die Lösung 48 Stunden am Wasserabscheider am Rückfluß. Danach schüttelte man die abgekühlte Lösung dreimal mit je 65 ml n NaOH und anschließend zweimal mit Wasser aus, trocknete die benzolische Lösung mit $Na_2SO_4$, filtrierte und dampfte sie im Vakuum ein. Der Rückstand wurde in 35 ml Methanol gelöst, woraufhin ein

Feststoff auskristallisierte. Dieser wurde abgesaugt und erwies sich als reines α-(Imidazol-1-yl)-2,4-dichlor-acetophenon. Man erhielt 6,32 g (62% vom Einsatz) dieses Ausgangs-Ketons. Die methanolische Mutterlauge wurde im Vakuum eingedampft. Der Rückstand wurde an einer Kieselgel S-$CH_2Cl_2$-Säule (⌀ 2,1 cm, Höhe 40 cm) wie im Beispiel 14 beschrieben chromatographiert. Die DC-einheitlichen Fraktionen (DC Vergleich mit gemäß Beispiel 3 hergestelltem Produkt) wurden vereinigt und im Vakuum eingedampft. Man erhielt 0,84 g (4,4% d.Th.) DC reines cis-trans-2-(2,4-Dichlorphenyl)-2-(imidazol-1-ylmethyl)-4-(benzothiazol-2-ylthiomethyl)-1,3-dioxolan als hochviskoses Öl,
Analyse: Gef. C 51,6, H 3,4, N 8,2%.

Beispiele für das Herstellungsverfahren Variante D)

Beispiel 26

Eine Lösung von 2,45 g (5 mMol) 2-S,(R)-(2,4-Dichlorphenyl)-2-(imidazol-1-ylmethyl)-4-R,(S)-(4-piperazino-phenoxymethyl)-1,3-dioxolan (cis-Form) und 1,01 g (5 mMol) 5-Chlor-2-chlormethyl-benzoxazol in 20 ml abs. DMF wurde auf 80°C unter Schutz gegen Luftfeuchtigkeit erwärmt und nach 10 Min. mit 115 mg feinpulverisiertem Kaliumcarbonat unter Rühren versetzt. Nach weiteren 25 Min. gab man weitere 115 mg pulverisiertes $K_2CO_3$ und nach weiteren 60 Min. eine 3. Portion von 115 mg pulverisiertem $K_2CO_3$ zu (insgesamt 345 mg (2,5 mMol) $K_2CO_3$). Anschließend rührte man 3 Std. bei 80°C nach, destillierte das DMF im Ölpumpenvakuum am Rotationsverdampfer ab und nahm den Rückstand in 30 ml Wasser und 100 ml $CH_2Cl_2$ auf. Nach Trennung der Phasen wurde die wäßrige noch zweimal mit $CH_2Cl_2$ extrahiert. Die vereinigten $CH_2Cl_2$-Extrakte wurden getrocknet, filtriert und im Vakuum eingedampft. Der Rückstand (3,6 g) wurde durch Säulenchromatographie an Kieselgel S/$CH_2Cl_2$ wie im Beispiel 14 beschrieben gereinigt. Man erhielt 2,75 g (84% d.Th.) reines Cis-2-(2,4-Dichlorphenyl)-2-(imidazol-1-ylmethyl)-4-[4-(4-(5-chlor-benzoxazol-2-ylmethyl)-piperazin-1-yl)-

phenoxymethyl]-1,3-dioxolan als hochviskoses Öl; Analyse: Gef.: C 58,6, H 4,7, N 10,6,%. (vgl. Beispiel 16, Tab. 4, Nr. 4.4).

Beispiel 27

Nach der gleichen Arbeitsweise wie im Beispiel 26 beschrieben, ausgehend von IXa oder IXb (vgl. Tabelle 5) und jeweils der entsprechenden Chlor-Verbindung der Formel Xa oder XIa wurden die in der Tabelle 5 aufgeführten Verbindungen der Formel I nach Verfahrensvariante D) hergestellt.

TABELLE 5

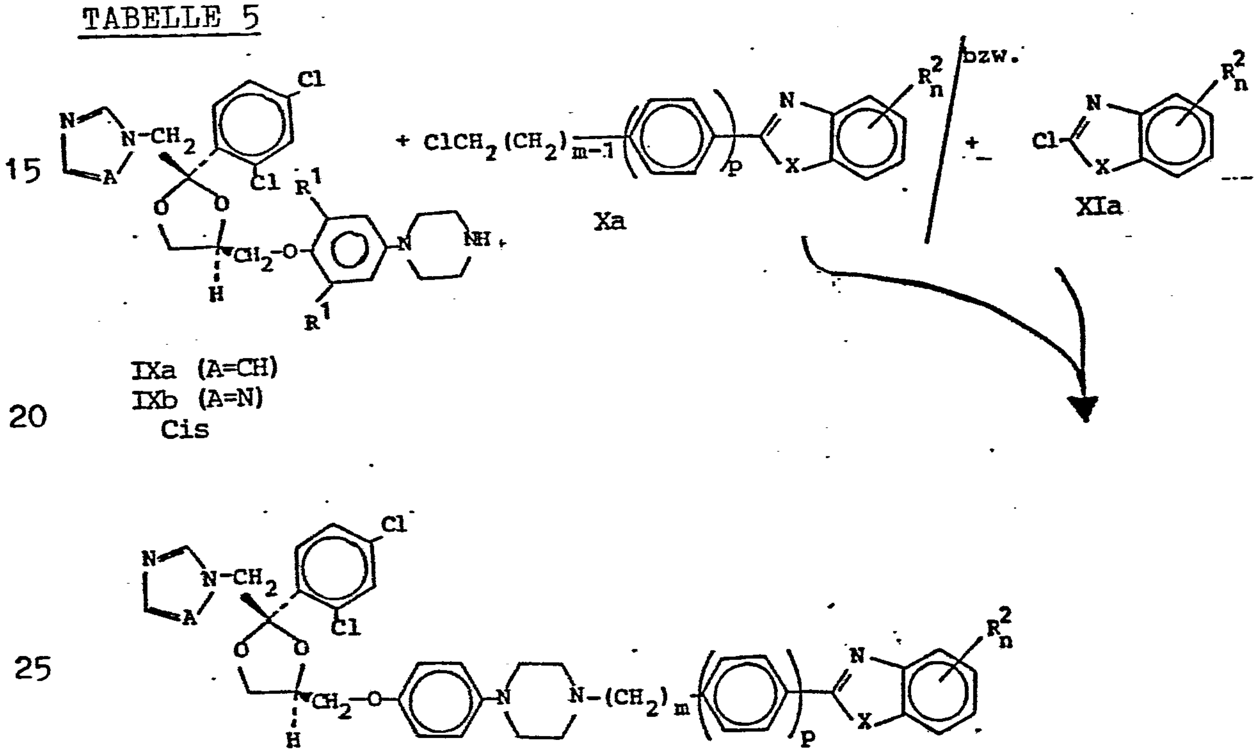


IXa (A=CH)
IXb (A=N)
Cis

Cis I (L=1, Ar= 2,4-$Cl_2$-$C_6H_3$-, g=0)

| Verb. Nr | A | m | p | X | $R^2$ | n | Fp. [°C] | Analyse % Gef. | Ber. | vgl. Tab.Nr.bzw.Beisp. |
|---|---|---|---|---|---|---|---|---|---|---|
| 5.1 | CH | 1 | 0 | 0 | 5,6-$(CH_3)_2$ | 2 | 156-7 | C 62,8<br>H 5,2<br>N 10,7 | | 4 4.2 |

TABELLE 5, Forts.

| Verb. Nr | A | m | p | X | $R^2$ | n | Fp. [°C] | Analyse Gef. | % Ber. | vgl. Tab.Nr.bzw.Beisp. |
|---|---|---|---|---|---|---|---|---|---|---|
| 5.2 | N | 1 | 0 | 0 | 5-Cl | 1 | - | C 56,8<br>H 4,2<br>N 12,5 | 4 | 4.5 |
| 5.3 | CH | 3 | 0 | S | - | 0 | - | C 61,1<br>H 5,2<br>N 10,3 | 4 | 4.13 |
| 5.4 | CH | 0 | 0 | S | 6-$OC_2H_5$ | 1 | 172/3 | C 59,2<br>H 5,0<br>N 10,2 | 3 | 3.6 |
| 5.5 | N | 0 | 0 | S | 6-$OC_2H_5$ | 1 | 173/4 | C 57,3<br>H 4,8<br>N 12,3 | 3 | 3.7 |
| 5.6 | CH | 1 | 1 | 0 | - | 0 | - | C 65,4<br>H 5,1<br>N 9,9 | | 17 |
| 5.7 | N | 1 | 1 | S | - | 0 | 164/5 | C 62,0<br>H 4,6<br>N 11,6 | | 18 |

Beispiel 28

Nach im Prinzip gleicher Arbeitsweise wie im Beispiel 26 beschrieben, ausgehend von 5 mMol IXa und 5 mMol 5-Chlor-2-chlormethyl-benzoxazol, jedoch in 50 ml abs. Acetonitril, anstelle von DMF als Lösungsmittel, wurden nach chromatographischer Reinigung 2,20 g (67,2% d. Th.) Cis-2-(2,4-Dichlorphenyl)-2-(imidazol-1-ylmethyl)-4-[4-(4-(5-chlor-benzoxazol-2-yl-

methyl)-piperazin-1-yl)-phenoxymethyl]-1,3-dioxolan (Öl) erhalten.

Beispiele für die Herstellung von Verbindungen der Formel IIIa

Beispiel 29

Eine Mischung von 5,11 g (15 mMol) 1-(4-Hydroxyphenyl)-piperazin-dihydrobromid, 2,46 g (16 mMol) 2-Chlorbenzoxazol und 2,18 g (15,8 mMol) pulverisiertem $K_2CO_3$ in 55 ml abs. N,N-Dimethylformamid wurde auf 85°C erwärmt und unter Rühren nach 10 Min., nach weiteren 25 Min. und nach weiteren 60 Min. mit jeweils 345 mg pulverisiertem $K_2CO_3$ unter Stickstoff-Überlagerung versetzt (insgesamt Zusatz von 1,035 g (7,5 mMol) $K_2CO_3$). Man rührte 3 Std. bei 85°C nach, destillierte im Ölpumpenvakuum am Rotationsverdampfer das DMF weitgehend ab und vermischte den kristallinen Rückstand mit 50 ml Wasser und schüttelte diese Mischung 15 Min.. Danach wurde abgesaugt, der Filterrückstand mit Wasser nachgewaschen und im Vakuum getrocknet. Man erhielt 4,30 g (97% d. Th.) reines 1-(4-Hydroxyphenyl)-4-(benzoxazol-2-yl)-piperazin, Fp. 161-3°C,

| | | | | | |
|---|---|---|---|---|---|
| Analyse: | Ber. | C | 69,13 | Gef. C | 69,1 % |
| $C_{17}H_{17}N_3O_2$ | | H | 5,80 | H | 5,7 |
| (MG 295,35) | | N | 14,23 | N | 14,1 |

Beispiel 30

Nach der gleichen Arbeitsweise wie im Beispiel 29 beschrieben wurden ausgehend von einer Verbindung der Formel XVI und jeweils dem entsprechenden 2-Chlorbenzazol der Formel XIa die in der Tabelle 6 aufgeführten Verbindungen der Formel IIIa hergestellt.

TABELLE 6

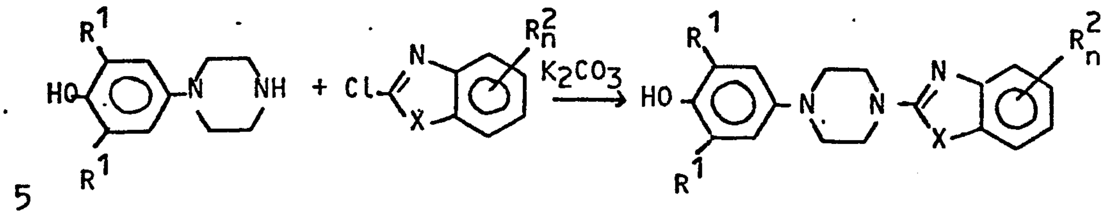


XVI XIa IIIa (m und p=0)

| Verb. Nr. | $R^1$ | X | $R^2$ | n | Ausbeute [%] | Fp. [°C] | Summenformel (MG) | Analyse Ber. | % Gef. |
|---|---|---|---|---|---|---|---|---|---|
| 6.1 | H | O | 6-Cl | 1 | 93 | 195-6 | $C_{17}H_{16}ClN_3O_2$ (329,80) | C 61,91<br>H 4,89<br>N 12,74 | 61,8<br>4,8<br>12,6 |
| 6.2 | H | O | 5-$CH_3$ | 1 | 83 | 205-6 | $C_{18}H_{19}N_3O_2$ (309,37) | C 69,88<br>H 6,19<br>N 13,58 | 69,8<br>6,2<br>13,4 |
| 6.3 | $CH_3$ | O | 5-$CH_3$ | 1 | 92 | 202-3 | $C_{20}H_{23}N_3O_2$ (337,43) | C 71,19<br>H 6,87<br>N 12,45 | 71,0<br>6,8<br>12,2 |
| 6.4 | H | S | 6-Cl | 1 | 98 | 183-4 | $C_{17}H_{16}ClN_3OS$ (345,86) | C 59,04<br>H 4,66<br>N 12,15 | 59,0<br>4,7<br>12,0 |
| 6.5 | H | S | 6-$OC_2H_5$ | 1 | 61 | 189-90 | $C_{19}H_{21}N_3O_2S$ (355,47) | C 64,20<br>H 5,96<br>N 11,82 | 63,5<br>5,9<br>11,6 |
| 6.6 | $CH_3$ | S | 5-Cl<br>6-$CH_3$ | 2 | | 211-2 | $C_{20}H_{22}ClN_3OS$ (387,94) | C 61,92<br>H 5,72<br>N 10,83 | 61,7<br>5,7<br>10,5 |

Beispiel 31

Eine Mischung von 6,06 g (34 mMol) 1-(4-Hydroxyphenyl)-piperazin und 5,36 g (32 mMol) 2-Chlormethyl-benzoxazol in 100 ml abs. DMF wurde auf 80°C erwärmt und unter Rühren (unter Stickstoffüberlagerung) nach 10 Min., nach weiteren 25 Min. und nach weiteren 60 Min. mit jeweils 737 mg pulverisiertem $K_2CO_3$ versetzt (insgesamt Zusatz von 2,21 g (16 mMol) $K_2CO_3$). Man rührte 3 Std. bei 80°C nach, destillierte im Vakuum das DMF weitgehend ab und nahm den Rückstand in 200 ml Ether und 100 ml Wasser auf. Nach Phasentrennung wurde die wäßrige Phase noch zweimal mit Ether extrahiert. Die vereinigten Etherextrakte wurden getrocknet, filtriert und im Vakuum eingedampft. Den Rückstand nahm man in Diisopropylether auf, wonach Kristallisation erfolgte. Die kristalline Substanz wurde abgesaugt. Man erhielt 7,10 g (71,7% d.Th.) 1-(4-Hydroxyphenyl)-4-(benzoxazol-2-ylmethyl)-piperazin, Fp. 155-56°C, Analyse: $C_{18}H_{19}N_3O_2$ (MG 309,37) Ber. C 69,88, H 6,19, N 13,58; Gef. C 69,9, H 6,0, N 13,3%.

Beispiel 32

Nach der gleichen Arbeitsweise wie im Beispiel 31 beschrieben wurden ausgehend von einer Verbindung der Formel XVI und jeweils der entsprechenden Verbindung der Formel Xa die in der Tabelle 7 aufgeführten Verbindungen der Formel IIIa hergestellt. Sofern nach Abdampfen des DMF aus der Reaktionsmischung ein fester, kristalliner Rückstand verblieb, wurde dieser gemäß der Arbeitsweise wie im Beispiel 29 beschrieben weiter aufgearbeitet .Das war bei der Mehrzahl der in der Tabelle 7 zusammengefaßten Beispiele der Fall. Verblieb an besagter Stelle ein teils fester, teils zähöliger Rückstand, dann wurde wie in Beispiel 31 beschrieben weiter aufgearbeitet.

Als Extraktionsmittel wurden Ether (wie im Beispiel 31) oder Methylenchlorid verwendet. Sofern primär in Gegenwart von Wasser kristallin isolierte Verbindungen der Formel IIIa laut DC nicht ganz rein anfielen, wurden deren Suspensionen in Ethylacetat 2-10 Min. am Rückfluß gekocht, abgekühlt und die kristalline Verbindung durch Absaugen isoliert. Bei Verwendung von Verbindungen Xa mit m=3 wurden der Reaktionsmischung zusätzlich 30 Mol% pulverisiertes Natriumiodid zugesetzt.

TABELLE 7

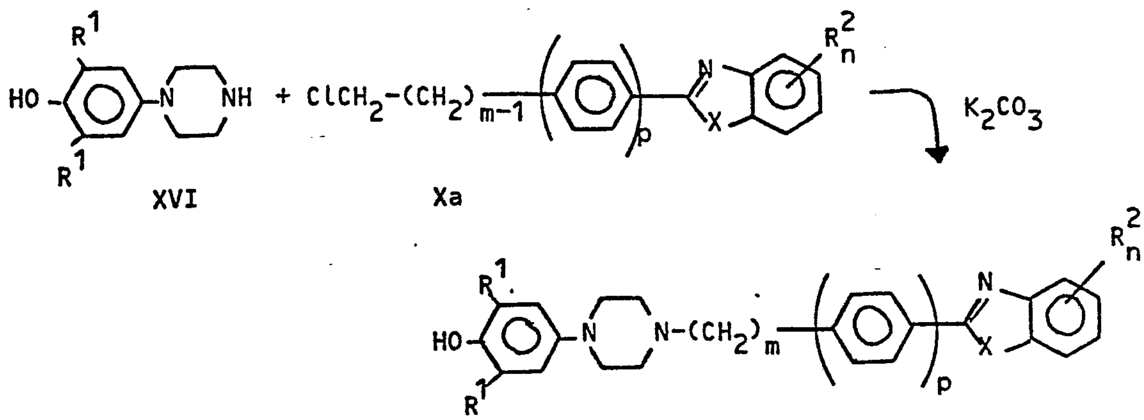


XVI Xa

IIIa

| Verb. Nr. | $R^1$ | m | p | X | $R^2$ | n | Ausbeute [%] | Fp. [°C] | Summenformel (MG) | Analyse | Ber. | % Gef. |
|---|---|---|---|---|---|---|---|---|---|---|---|---|
| 7.1 | H | 1 | 0 | 0 | 5-Cl | 1 | 95 | 145-6 | $C_{18}H_{18}ClN_3O_2$ (343,82) | C | 62,88 | 62,6 |
| | | | | | | | | | | H | 5,28 | 5,2 |
| | | | | | | | | | | Cl | 10,31 | 10,6 |
| | | | | | | | | | | N | 12,22 | 12,2 |
| 7.2 | H | 1 | 0 | 0 | 5,6-$(CH_3)_2$ | 2 | 92 | 191-2 | $C_{20}H_{23}N_3O_2$ (337,43) | C | 71,19 | 69,4 |
| | | | | | | | | | | H | 6,87 | 6,8 |
| | | | | | | | | | | N | 12,45 | 12,1 |
| 7.3 | H | 1 | 0 | S | - | 0 | | 175-6 | $C_{18}H_{19}N_3OS$ (325,44) | C | 66,43 | 66,0 |
| | | | | | | | | | | H | 5,88 | 6,1 |
| | | | | | | | | | | N | 12,91 | 12,8 |

TABELLE 7, Forts.

| Verb. Nr. | $R^1$ | m | p | X | $R^2$ | n | Ausbeute [%] | Fp. [°C] | Summenformel (MG) | Analyse | Ber. % | Gef. % |
|---|---|---|---|---|---|---|---|---|---|---|---|---|
| 7.4 | H | 1 | 0 | 0 | 4,5- 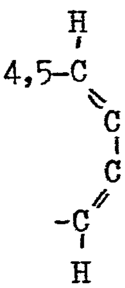 | 2 | 89 | 208-9 | $C_{22}H_{21}N_3O_2$ | C | 73,52 | 73,2 |
| | | | | | | | | | (359,43) | H | 5,89 | 5,7 |
| | | | | | | | | | | N | 11,69 | 11,7 |
| 7.5 | $CH_3$ | 1 | 0 | 0 | - | 0 | 70 | 118-9 | $C_{20}H_{23}N_3O_2$ | C | 71,19 | 70,7 |
| | | | | | | | | | (337,43) | H | 6,87 | 6,6 |
| | | | | | | | | | | N | 12,45 | 12,2 |
| 7.6 | $CH_3$ | 1 | 0 | S | - | 0 | 55 | 143-4 | $C_{20}H_{23}N_3OS$ | C | 67,96 | 67,7 |
| | | | | | | | | | (353,49) | H | 6,56 | 6,7 |
| | | | | | | | | | | N | 11,89 | 11,8 |
| | | | | | | | | | | S | 9,07 | 9,0 |
| 7.7 | H | 3 | 0 | 0 | - | 0 | 83 | 175-6 | $C_{20}H_{23}N_3O_2$ | C | 71,19 | 71,3 |
| | | | | | | | | | (337,43) | H | 6,87 | 7,0 |
| | | | | | | | | | | N | 12,45 | 12,4 |
| 7.8 | H | 3 | 0 | 0 | 5-Cl | 1 | 83 | 110-11 | $C_{20}H_{22}ClN_3O_2$ | C | 64,60 | 63,05 |
| | | | | | | | | | (371,88) | H | 5,96 | 6,0 |
| | | | | | | | | | | Cl | 9,53 | 9,7 |
| | | | | | | | | | | N | 11,30 | 10,95 |
| 7.9 | H | 3 | 0 | S | - | 0 | 38 | 127-8 | $C_{20}H_{23}N_3OS$ | C | 67,96 | 68,4 |
| | | | | | | | | | (353,49) | H | 6,56 | 6,6 |
| | | | | | | | | | | N | 11,89 | 12,2 |
| 7.10 | $CH_3$ | 3 | 0 | 0 | - | 0 | 77 | 121-2 | $C_{22}H_{27}N_3O_2$ | C | 72,30 | 72,0 |
| | | | | | | | | | (365,48) | H | 7,45 | 7,6 |
| | | | | | | | | | | N | 11,50 | 11,4 |

TABELLE 7, Forts.

| Verb. Nr. | $R^1$ | m | p | X | $R^2$ | n | Ausbeute [%] | Fp. [°C] | Summenformel (MG) | Analyse Ber. | % Gef. |
|---|---|---|---|---|---|---|---|---|---|---|---|
| 7.11 | $CH_3$ | 3 | 0 | S | - | 0 | 35 | 140-2 | $C_{22}H_{27}N_3OS$ (381,55) | C 69,26<br>H 7,13<br>N 11,01 | 69,4<br>6,9<br>10,7 |
| 7.12 | H | 1 | 1 | O | - | 0 | 90 | 235-6 | $C_{24}H_{23}N_3O_2$ (385,47) | C 74,78<br>H 6,01<br>N 10,90 | 73,8<br>5,8<br>10,4 |
| 7.13 | H | 1 | 1 | S | - | 0 | 94 | 234-5 | $C_{24}H_{23}N_3OS$ (401,54) | C 71,80<br>H 5,77<br>N 10,47 | 71,7<br>5,9<br>14,3 |

Beispiel 33

Zu einer Mischung von 1,78 g (10 mMol) 1-(4-Hydroxyphenyl)-piperazin, 2,48 g (13,5 mMol) 2-Chlormethyl-benzothiazol und 80 ml abs. 1,2-Dimethoxyethan wurde unter Rühren unter Rückflußkochen innerhalb von 15 Min. eine Lösung von 1,05 g Triethylamin in 20 ml abs. Dimethoxyethan zugetropft. Man rührte 5 Std. unter Rückfluß nach, verdampfte im Vakuum das Lösungsmittel, nahm den Rückstand in $CH_2Cl_2$/$H_2O$ auf, trennte die Phasen und extrahierte die wäßrige Phase dreimal mit $CH_2Cl_2$. Die vereinigten $CH_2Cl_2$-Extrakte wurden getrocknet, filtriert und eingedampft. Der kristalline Rückstand (3,2 g) wurde aus Methanol umkristallisiert. Man erhielt 2,64 g (81,2% d. Th.) reines 1-(4 - Hydroxyphenyl)-4-(benzothiazol-2-ylmethyl)-piperazin, Fp. 175-76°C.

Diese Synthese konnte auch mit 1-(4-Hydroxyphenyl)-piperazin-dihydrobromid ausgeführt werden. In diesem Fall wurden bei einem 10 mMol-Ansatz 3,10 g (30,7 mMol) Triethylamin verwendet und die Reaktionszeit wurde auf 18 Std.

ausgedehnt. Man erhielt etwa die gleiche Menge (2,6-2,7 g) reines 1-(4-Hydroxyphenyl)-4-(benzothiazol-2-ylmethyl)-piperazin wie bei der vorstehenden Ausführung mit der Base des Piperazin-Derivats.

Herstellung von Ausgangsstoffen für die Verfahrens-Variante C):

Beispiel 34

Eine Lösung von 10,04 g (60 mMol) 2-Mercaptobenzothiazol in 100 ml abs. DMF wurde (unter Kühlung) bei ca. 40°C portionsweise mit 1,80 g (60 mMol) 80%iger NaH-Öl-Dispersion versetzt und 15 Min. bei 40°C gerührt, wonach die Wasserstoffentwicklung beendet war. Man gab 6,63 g (5,02 ml, 60 mMol) 1-Chlor-2,3-dihydroxypropan und 0,30 g (2 mMol) pulverisiertes Natriumiodid zu, rührte 3 Std. bei 87°C und destillierte anschließend im Vakuum am Rotationsverdampfer das DMF weitgehend ab. Den öligen Rückstand nahm man in 300 ml $CH_2Cl_2$ auf und die Lösung wurde nacheinander zweimal mit je 80 ml 0,2 n NaOH und zweimal mit Wasser ausgeschüttelt, getrocknet, filtriert und im Vakuum eingedampft. Der verbleibende Rückstand, 12,5 g zähes Öl wurde an einer Kieselgel S/$CH_2Cl_2$-Säule (ø 2,0 cm, Höhe 38 cm) durch Elution mit $CH_2Cl_2$ und $CH_2Cl_2$/Ethanol 100:0,5 - 100:3 (v/v)-Mischungen chromatographiert. Die DC-einheitlichen Fraktionen wurden vereinigt und intensiv im Ölpumpenvakuum eingedampft.

Man erhielt 9,0 g (62,2 % d. Th.) 1-(Benzothiazol-2-ylthio)-2,3-dihydroxy-propan, eine Verbindung der Formel VIII (Z=S, L,m und p=0), als hochviskoses Öl, Analyse: $C_{10}H_{11}NO_2S_2$ (MG 241,34) Ber. C 49,77, H 4,59, N 5,80, S 26,57, Gef. C 48,8, H 4,6, N 5,7, S 26,3%.

Beispiel 35

Eine Mischung aus 108 g (1,17 Mol) wasserfreiem Glycerin und 1200 ml abs. 1,2-Dimethoxyethan wurde unter Kühlung und Rühren mit 7,0 g (0,233 Mol) 80%iger NaH-Öl-Dispersion versetzt, 6 Std. bei 50°C intensiv gerührt, anschließend

mit 36,72 g (0,18 Mol) 2,6-Dichlorbenzothiazol versetzt und 15 Std. am Rückfluß unter intensiver Rührung gekocht. Danach goß man bei Zimmertemperatur die Dimethoxyethan-Lösung von der viskosen Ölphase (Glycerin) ab und rührte nacheinander die Glycerinphase dreimal unter Rückfluß mit Dimethoxyethan aus. Die vereinigten Dimethoxyethan-Lösungen wurden im Vakuum eingedampft. Den öligen Rückstand nahm man in 1000 ml $CH_2Cl_2$ und 600 ml wäßriger $NH_4Cl$-Lösung auf, durchmischte intensiv und trennte die Phasen. Die $CH_2Cl_2$-Lösung wurde mit Wasser ausgewaschen, getrocknet, filtriert und im Vakuum eingedampft. Der teilweise kristalline Rückstand (28 g) wurde mit 45 ml Chloroform 10 Min. gekocht, abgekühlt und abgesaugt. Bei dem Filterrückstand (getrocknet 8,9 g) handelt es sich um 6-Chlor-2,3-dihydrobenzothiazol-2-on, das als Nebenprodukt gebildet wurde. Der Filtrat-Rückstand (19,1 g) wurde an einer Kieselgel S/$CH_2Cl_2$-Säule (ø 2,6 cm, Höhe 35 cm) chromatographiert. Man eluierte mit $CH_2Cl_2$ und $CH_2Cl_2$/Ethanol 100:0,5-100:5 (v/v)-Mischungen. Zuerst wurde neben unverändertem 2,6-Dichlorbenzothiazol eine weitere Menge 6-Chlor-2,3-dihydro-benzothiazol-2-on (insgesamt 11 g) eluiert. Anschließend eluierte man ca. 8 g hochangereichertes 1-(6-Chlor-benzothiazol-2-yloxy)-2,3-dihydroxypropan, das aus Acetonitril umkristallisiert wurde. Man erhielt 6,30 g (13,5% d.Th.) reines Produkt, Fp. 119-20°C,

| | | | | |
|---|---|---|---|---|
| Analyse: | Ber. | C 46,25 | Gef. | C 45,7% |
| $C_{10}H_{10}ClNO_3S$ | | H 3,88 | | H 3,6 |
| (MG 259,72) | | Cl 13,65 | | Cl 14,1 |
| | | N 5,39 | | N 5,4 |
| | | S 12,35 | | S 11,9 |

## Antimykotische Wirksamkeit der Verbindungen

Als Beispiel für die hohe lokale in vivo Wirksamkeit der erfindungsgemäßen Verbindungen werden Behandlungsergebnisse an experimentell mit Trichophyton mentagrophytes infizierten Labortieren angeführt.

Zur Feststellung der lokalen Wirksamkeit wurden jeweils zwei 450-500 g schwere Meerschweinchen (Stamm Pirbright white) mit 1,5 x $10^4$ Konidien/Tier in die Epidermis, verteilt auf 6 Infektionspunkte, infiziert.

Die Behandlung der Tiere erfolgte dermal ab dem 3. Tag nach der Infektion an 5 aufeinanderfolgenden Tagen durch Aufbringen einer 0,1%igen Präparatlösung auf 3 Infektionsstellen einer Rückenseite. Die andere Rückenseite wurde mit Vehikel ohne Präparat auf die gleiche Weise behandelt.

Neben den mit den erfindungsgemäßen Substanzen behandelten Tieren wurden zwei Tiere mit der Referenzsubstanz Terconazol behandelt, zwei Tiere blieben nach der Infektion unbehandelt.

Wie in Tabelle 8 ersichtlich, zeigten die erfindungsgemäßen Verbindungen eine deutlich stärkere Reduzierung der Mykosendurchmesser als das Standardpräparat Terconazol, d.h. der antimykotische Effekt der erfindungsgemäßen Verbindung war demjenigen von Terconazol bis zu 80% überlegen.

Tabelle 8

| Konzentration | Präparat Beispiel Nr. | Mykosen (Durchmesser in mm) Vehikelkontrollen | | | Präparat + Vehikel | | | Differenz |
|---|---|---|---|---|---|---|---|---|
| | | $x_1$ | $(s)$ 2) | $n$ 1) | $x_2$ | $(s)$ | $n$ 1) | $x_1 - x_2$ (%) |
| Dermal | | | | | | | | |
| 5 x 0,1 % | (16) 4.2 | 14,3 | (2,8) | 6 | 4,1 | (1,1) | 6 | 10,2 (156,9) |
| | (16) 4.10 | 15,0 | (2,5) | 6 | 4,1 | (0,9) | 6 | 10,9 (167,6) |
| | ( 2) 1.4 Base | 14,7 | (1,3) | 6 | 3,0 | (1,0) | 6 | 11,7 (180,0) |
| | Terconazol | 13,8 | (1,7) | 6 | 7,3 | (1,8) | 6 | 6,5 (100,0) |
| Kontrollen infizierte Tiere unbehandelt' | - | 14,6 | (3,9) | 12 | | - | | - |

1) n = Anzahl Meßwerte

2) (s)= Standardabweichung

Als Beispiel für die hohe orale und parenterale in-vivo-Wirksamkeit der erfindungsgemäßen Verbindungen werden Behandlungsergebnisse an experimentell mit Candida albicans infizierten Labortieren angeführt.

Zur Feststellung der oralen und parenteralen Wirksamkeit wurden Gruppen von je 5 18-20 g schweren Mäusen (Stamm HOE: NMRKF; SPF 71) mit 2 . $10^6$ Keimen/Tier infiziert.

Die Behandlung der Tiere erfolgte oral bzw. subcutan in 8 gleichen Einzeldosen zu je 30 mg/kg bzw. 10 mg/kg Körpergewicht (-24/-18/-2h/+2/24/30/48/54h).

Neben der mit den erfindungsgemäßen Substanzen I behandelten Gruppe von 5 Tieren wurde zum Vergleich eine Gruppe von ebenfalls 5 Tieren mit der Referenzsubstanz Ketoconazol behandelt. Eine Kontrollgruppe von 10 Tieren blieb nach der Infektion unbehandelt.

Wie aus Tabelle 9 hervorgeht, fand sich bei den erfindungsgemäßen Verbindungen eine bis um das Doppelte verlängerte Überlebenszeit der Tiere nach der Infektion, verglichen mit dem derzeitigen Standardpräparat Ketoconazol.

Tabelle 9

| Dosis | Präparat Beispiel Nr. | Anzahl Tiere | Überlebenszeiten Tage nach Infektion | | | | | $\bar{x}$ Tage | Überlebenszeit in % (Standardpräp. = 100 %) |
|---|---|---|---|---|---|---|---|---|---|
| oral 8 x 30 mg/kg | (13) 3.3 | 5 | 11 | 11 | 14 | 14 | 14 | 12,8 | 177,7 |
| | (16) 4.2 | 5 | 8 | 9 | 9 | 10 | 14 | 10,0 | 138,8 |
| | (16) 4.4 | 5 | 6 | 6 | 10 | 14 | 14 | 10,0 | 138,8 |
| | Ketoconazol | 5 | 6 | 7 | 7 | 8 | 8 | 7,2 | 100 |
| oral 8 x 10 mg/kg | (13) 3.3 | 5 | 9 | 10 | 11 | 13 | 13 | 11,2 | 180,6 |
| | (16) 4.2 | 5 | 5 | 6 | 6 | 7 | 7 | 6,2 | 106,8 |
| | (16) 4.4 | 5 | 5 | 6 | 6 | 7 | 8 | 6,4 | 110,3 |
| | Ketoconazol | 5 | 5 | 6 | 6 | 6 | 6 | 5,8 | 100 |
| subcutan 8 x 30 mg/kg | (13) 3.3 | 5 | 11 | 12 | 12 | 14 | 14 | 12,6 | 153,6 |
| | (16) 4.2 | 5 | 8 | 9 | 11 | 13 | 14 | 11,0 | 134,1 |
| | (16) 4.4 | 5 | 8 | 9 | 10 | 12 | 14 | 10,6 | 129,2 |
| | Ketoconazol | 5 | 7 | 7 | 8 | 8 | 11 | 8,2 | 100 |
| subcutan 8 x 10 mg/kg | (13) 3.3 | 5 | 10 | 11 | 11 | 11 | 13 | 11,2 | 233,3 |
| | (16) 4.2 | 5 | 7 | 8 | 8 | 11 | 11 | 9,0 | 187,5 |
| | (16) 4.4 | 5 | 5 | 7 | 7 | 7 | 8 | 6,8 | 141,6 |
| | Ketoconazol | 5 | 4 | 4 | 5 | 5 | 6 | 4,8 | 100 |
| Kontrollen infizierte Tiere unbehandelt | - | 10 | 1<br>2 | 1<br>2 | 1<br>3 | 2<br>3 | 2<br>3 | 2,0 | 30,3 |

## Patentansprüche

1. Verbindungen der Formel I

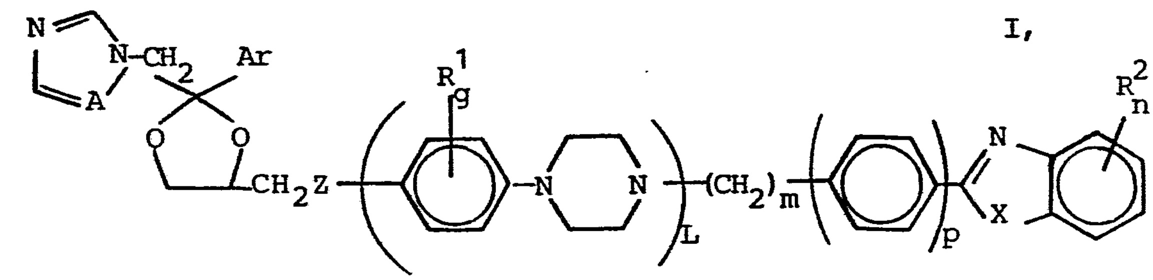

I,

in der bedeuten:

A CH oder N,

Ar Naphthyl, Thienyl, Halothienyl oder eine unsubstituierte oder eine bis zu 3 Substituenten tragende Phenylgruppe, wobei die Substituenten gleich oder verschieden sein können und F, Cl, Br, J, $CF_3$, $OCH_3$, $OC_2H_5$, $CH_3$, $C_2H_5$, $C_6H_5$ oder Phenoxy bedeuten,

Z O oder, falls gleichzeitig L, m und n Null bedeuten, O oder S,

$R^1$ $C_1$-$C_3$-Alkyl, F oder Cl,

g 0, 1 oder 2,

L 0 oder 1,

m 0, 1, 2, 3 oder 4,

p 0 oder, falls m ungleich 0 ist oder falls gleichzeitig L und m = 0 sind, 0 oder 1 und

X O oder falls m ungleich 0 ist oder gleichzeitig L, m und p = 0 sind oder falls gleichzeitig m und p = 0 und n ungleich 0 sind, O, S oder N-$R^3$, wobei $R^3$ H, $C_1$-$C_4$-Alkyl, Phenyl oder Benzyl bedeutet, und

$R^2$ unabhängig voneinander $C_1$-$C_4$-Alkyl, $C_1$-$C_4$-Alkoxy, F, Cl, Br, J, $SCH_3$, CO-$C_6H_5$, $CF_3$, $COOCH_3$, $COOC_2H_5$ oder $NO_2$ und

n 0, 1 oder 2 oder, falls n=2 ist,

$R^2$ zusätzlich eine -CH=CH-CH=CH-Gruppe , die mit dem Phenylring zusammen einen Naphthyl-Rest bildet, oder,
falls gleichzeitig p = 0 ist und n = 1 ist,
$R^2$ zusätzlich eine unsubstituierte oder im Phenylrest bis zu 2 gleiche oder verschiedene Substituenten tragende Phenoxygruppe darstellt, wobei die Substituenten F, Cl, Br, $OCH_3$, $CH_3$ oder $C_2H_5$ bedeuten,

sowie deren physiologisch verträgliche Säureadditionssalze.

2. Verbindung der Formel I nach Anspruch 1, in der mindestens einer der Substituenten und Indices bedeuten:

A CH oder N,
Ar eine durch 1 oder 2 F- oder Cl-Atome substituierte Phenylgruppe,
Z O oder,
falls gleichzeitig L, m und p Null bedeuten,
O oder S,
$R^1$ $CH_3$ oder $C_2H_5$,
g 0 oder 2,
L 0 oder 1,
m 0, 1, 2 oder 3,
p 0 oder, falls m = 1 ist, 0 oder 1,
X O, oder
falls m = 1, 2 oder 3 ist, oder falls gleichzeitig L und m = 0 sind, oder falls gleichzeitig m = 0 ist und n = 1 oder 2 ist, O oder S,
$R^2$ unabhängig voneinander $C_1$-$C_4$-n-Alkyl, $C_1$-$C_4$-Alkoxy, F, Cl, Br oder, falls p = 0 und n = 1 ist, $OC_6H_5$ und
n 0, 1 oder 2.

3. Verbindung der Formel I nach Anspruch 1, in der mindestens einer der Substituenten und Indices bedeuten:

A CH oder N,
Ar 2,4-Dichlorphenyl,
Z O,
$R^1$ $CH_3$,

g 0 oder 2,
L 0 oder 1,
m 0, 1, 2 oder 3,
p 0 oder, falls m = 1 ist, 0 oder 1,
X 0 oder, falls m = 1, 2 oder 3 ist oder falls gleichzeitig L und m = 0 sind, 0 oder S,
$R^2$ unabhängig voneinander $CH_3$, $C_2H_5$, $C_1$-$C_4$-Alkoxy, F, Cl oder Br und
n 0, 1 oder 2.

4. Verbindung der Formel I nach Anspruch 1, dadurch gekennzeichnet, daß gleichzeitig L, m und p Null oder L Null und gleichzeitig m und p 1 und jeweils X 0 oder S und n Null oder 1 bedeuten und $R^2$ $CH_3$, $C_2H_5$, $C_1$-$C_4$-Alkoxy, F, Cl oder Br bedeuten.

5. Verbindung I nach Anspruch 1, dadurch gekennzeichnet, daß der Azolylmethylrest und die 4-ständige Piperazinophenoxymethylgruppe am Dioxolanring in cis-Stellung stehen.

6. Verfahren zum Herstellen von Verbindungen der Formel I nach Anspruch 1, dadurch gekennzeichnet, daß man
A) eine Verbindung der Formel II,

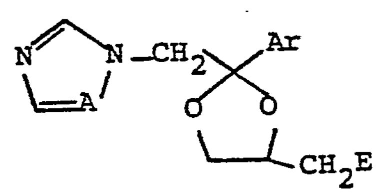

II,

in der
A und Ar die zu Formel I angegebenen Bedeutungen haben und
E Cl, Br, J oder Acyloxy, wie Acetoxy, Trifluoracetyloxy, Benzoyloxy, Nitrobenzoyloxy, $C_1$-$C_3$-Alkylsulfonyloxy wie Methansulfonyloxy, oder Arylsulfonyloxy, wie Benzol-, Nitrobenzol-, Brombenzol- oder Toluolsulfonyloxy, bedeutet,
mit einer Verbindung der Formel III,

0237964
HOE 86/F 062

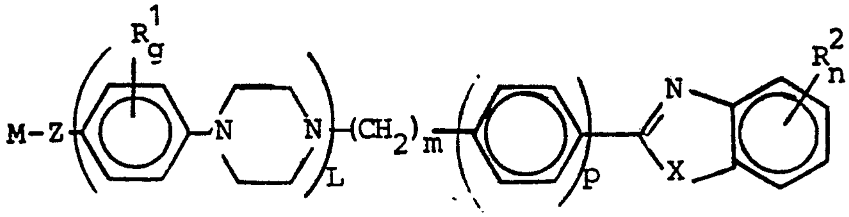 III,

in der

Z O oder, falls gleichzeitig L, m und p Null bedeuten, S bedeutet und

M H, ein Alkali- oder Erdalkalimetall, insbesondere Li, Na oder K, oder $NH_4$ bedeutet und

$R^1$, g, L, m,p, X, $R^2$ und n die zu Formel I angegebenen Bedeutungen haben, umsetzt, oder daß man

B) eine Verbindung der Formel IV,

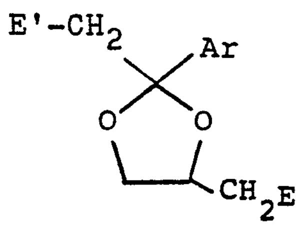 IV

in der
Ar die zu Formel I und E und E' die zu Formel II für E angegebenen Bedeutungen haben,
zunächst mit einer Verbindung der Formel III

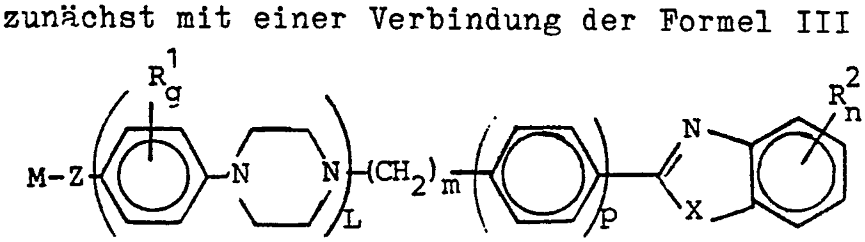 III

umsetzt und hierbei eine Verbindung der Formel V herstellt,

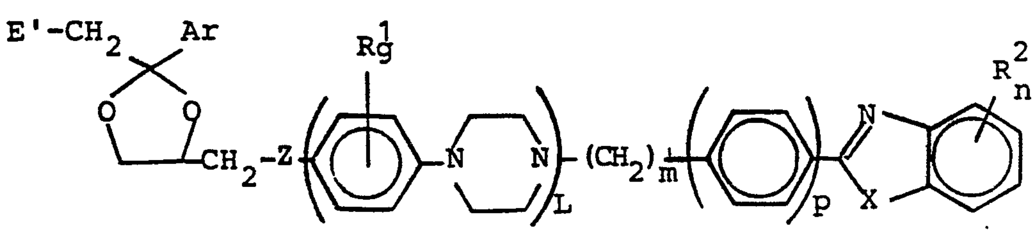 V

in der Z O oder, falls gleichzeitig L, m und p Null bedeuten, S bedeutet, und Ar, $R^1$, g, L, m, p, X, $R^2$ und n die zu Formel I und E' die zu Formel II für E angegebenen Bedeutungen haben, und anschließend eine Verbindung der Formel V mit einer Verbindung der Formel VI umsetzt,

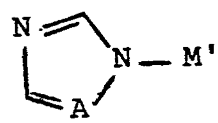

VI

in der

A CH oder N und

M' H, ein Alkali- oder Erdalkalimetall oder $Si(CH_3)_3$ bedeutet,

oder daß man

C) eine Verbindung der Formel VII,

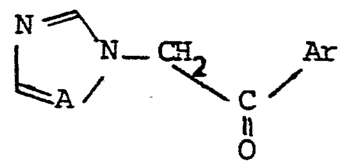

VII

in der A und Ar die zu Formel I angegebenen Bedeutungen haben, mit einem 1,2-Diol der Formel VIII

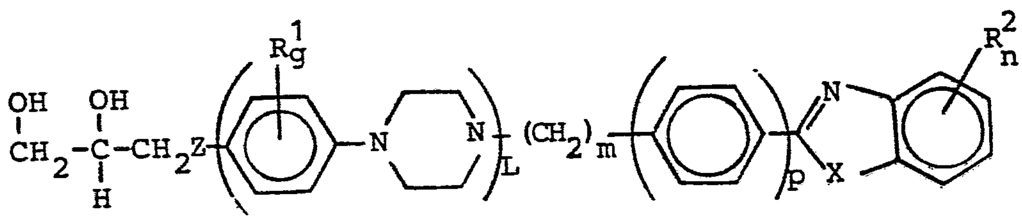

VIII

in der Z, $R^1$, g, L, m, p, X, $R^2$ und n die zu Formel I angegebenen Bedeutungen haben, umsetzt,

oder daß man

D) eine Verbindung der Formel IX,

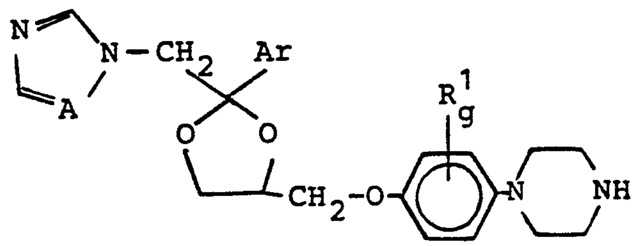

IX

in der A, Ar, $R^1$ und g die zu Formel I angegebenen Bedeutungen haben, oder ein Salz dieser Verbindung, entweder mit einer Verbindung der Formel X,

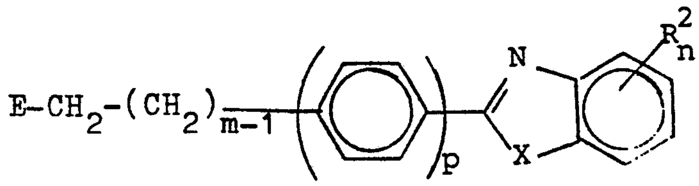

X

in der E Cl, Br, J, Acyloxy, wie Acetyloxy, Trifluoracetyloxy, Benzoyloxy, Nitrobenzoyloxy, $C_1$-$C_3$-Alkylsulfonyloxy, wie Methansulfonyloxy, oder Arylsulfonyloxy, wie Benzol-, Nitrobenzol-, Brombenzol- oder Toluolsulfonyloxy, und

m 1, 2, 3 oder 4 bedeutet, und

p, X, $R^2$ und n die zu Formel I angegebenen Bedeutungen haben,

oder mit einer Verbindung der Formel XI,

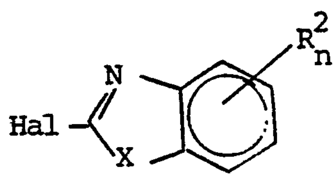

XI

in der

Hal Cl, Br, oder J, vorzugsweise Cl,

X O, oder,
falls n ungleich O ist, O, S oder $NR^3$, wobei $R^3$ H, $C_1$-$C_4$-Alkyl, Phenyl oder Benzyl bedeutet, bedeuten und

$R^2$ und n die zu Formel I angegebenen Bedeutungen haben, umsetzt, oder daß man

E) eine Verbindung der Formel IIa,

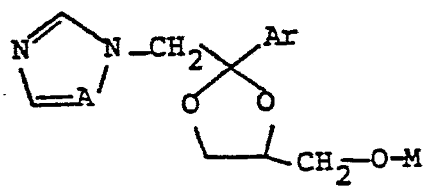

IIa

in der A und Ar die zu Formel I angegebenen Bedeutungen haben und

M H, ein Alkali- oder Erdalkalimetall, insbesondere Li, Na oder K bedeutet,

mit einer Verbindung der Formel X,

in der

E, m, p, X, $R^2$ und n die zu Formel X angegebenen Bedeutungen haben,

unter Bildung einer Verbindung der Formel I, in der Z O bedeutet und A, Ar, $R^1$, g, m, p, X, $R^2$ und n die angegebenen Bedeutungen haben und L = O bedeutet, umsetzt, oder daß man

F) eine Verbindung der Formel IIa,

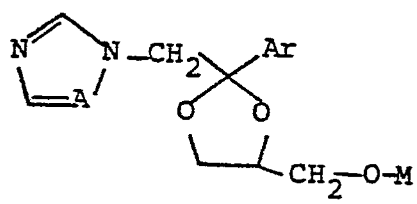

IIa

in der A und Ar die zu Formel I angegebenen Bedeutungen haben und

M H, ein Alkali- oder Erdalkalimetall, insbesondere Li, Na oder K, bedeutet,

mit einer Verbindung der Formel XI,

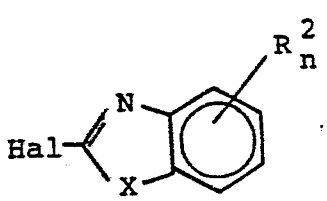

XI

in der

Hal Cl, Br oder J, vorzugsweise Cl, bedeutet und X, $R^2$ und n die zu Formel I angegebenen Bedeutungen haben, unter Bildung einer Verbindung der Formel I, in der Z O bedeutet und A, Ar, $R^1$, g, X, $R^2$ und n die angegebenen Bedeutungen haben und L, m und p gleichzeitig O bedeuten, umsetzt

und gegebenenfalls die nach Weg A)-F) erhaltenen Verbindungen der Formel I mit anorganischen oder organischen Säuren in ihre physiologisch verträglichen Salze überführt.

7. Verbindung der Formel IIIa

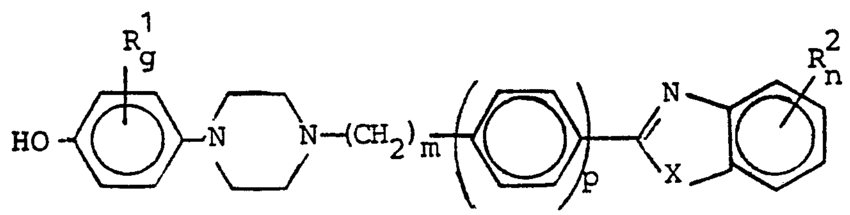

IIIa

in der bedeuten:

$R^1$ $C_1$-$C_3$-Alkyl, F oder Cl,

g 0, 1 oder 2,

m 0, 1, 2, 3 oder 4,

p 0 oder
falls m ungleich 0 ist, 0 oder 1 und

X O oder,
falls m ungleich 0 ist oder falls gleichzeitig m und p=0 und n ungleich 0 sind, O, S oder N-$R^3$, wobei

$R^3$ H, $C_1$-$C_4$-Alkyl, Phenyl oder Benzyl bedeutet, und

$R^2$ unabhängig voneinander $C_1$-$C_4$-Alkyl, $C_1$-$C_4$-Alkoxy, F, Cl, Br, J, $SCH_3$, CO-$C_6H_5$, $CF_3$ oder $NO_2$ und

n 0, 1 oder 2

oder, falls n = 2 ist,

$R^2$ zusätzlich eine -CH=CH-CH=CH-Gruppe, die mit

dem Phenylring zusammen einen Naphthyl-Rest bildet,

oder, falls p = 0 ist und n = 1 ist,

$R^2$ zusätzlich eine unsubstituierte oder im Phenylrest bis zu 2 gleiche oder verschiedene Substituenten tragende Phenoxygruppe, wobei die Substituenten F, Cl, Br, $OCH_3$, $CH_3$ oder $C_2H_5$ bedeuten,

sowie deren Säureadditionssalze.

8. Verfahren zum Herstellen einer Verbindung der Formel IIIa nach Anspruch 7, dadurch gekennzeichnet, daß man eine Verbindung der Formel XVI,

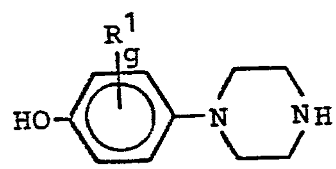

XVI

in der $R^1$ und g die zu Formel IIIa angegebenen Bedeutungen haben, oder ein Salz dieser Verbindung, entweder mit einer Verbindung der Formel X,

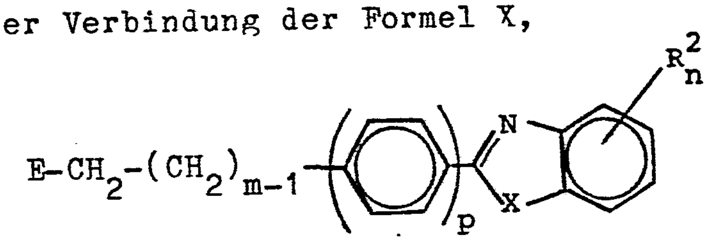

X

in der

E Cl, Br, J, Acyloxy, wie Acetoxy, Trifluoracetoxy, Benzoyloxy, Nitrobenzoyloxy, $C_1$-$C_3$-Alkylsulfonyloxy, wie Methansulfonyloxy, oder Arylsulfonyloxy, wie Benzol-, Nitrobenzol-, Brombenzol- oder Toluolsulfonyloxy und

m 1, 2, 3 oder 4 bedeutet, und

p, X, $R^2$ und n die zu Formel IIIa angegebenen Bedeutungen haben,

oder mit einer Verbindung der Formel XI,

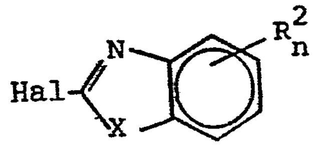

XI

in der

Hal Cl, Br oder J, vorzugsweise Cl,

X O, oder falls n ungleich Null ist, O, S oder N-$R^3$, wobei $R^3$ H, $C_1$-$C_4$-Alkyl, Phenyl oder Benzyl bedeutet, bedeuten und

$R^2$ und n die zu Formel IIIa angegebenen Bedeutungen haben, umsetzt,

und gegebenenfalls die erhaltenen Verbindungen der Formel IIIa mit anorganischen oder organischen Säuren in ihre Säureadditionssalze überführt.

9. Verwendung einer Verbindung der Formel I nach Anspruch 1 als Antimykotikum.

10. Verwendung einer Verbindung der Formel I nach Anspruch 1 zur Herstellung eines antimykotisch wirkenden Arzneimittels.

11. Arzneimittel mit antimykotischer Wirkung, gekennzeichnet durch einen wirksamen Gehalt einer Verbindung I nach Anspruch 1.

12. Verfahren zum Behandeln von Mykosen, dadurch gekennzeichnet, daß man eine wirksame Menge einer Verbindung I nach Anspruch 1 zusammen mit pharmazeutisch geeigneten Trägerstoffen appliziert.

Patentansprüche Griechenland, Spanien und Österreich:

1. Verfahren zum Herstellen von Verbindungen der Formel I

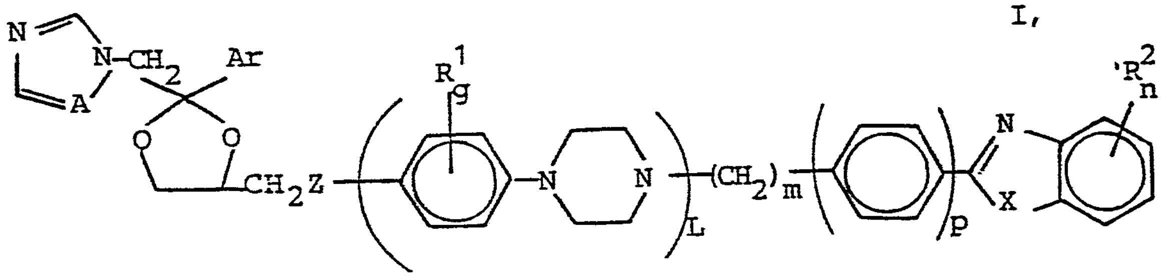

I,

in der bedeuten:

A CH oder N,

Ar Naphthyl, Thienyl, Halothienyl oder eine unsubstituierte oder eine bis zu 3 Substituenten tragende Phenylgruppe, wobei die Substituenten gleich oder verschieden sein können und F, Cl, Br, J, $CF_3$, $OCH_3$, $OC_2H_5$, $CH_3$, $C_2H_5$, $C_6H_5$ oder Phenoxy bedeuten,

Z O oder, falls gleichzeitig L, m und n Null bedeuten, O oder S,

$R^1$ $C_1$-$C_3$-Alkyl, F oder Cl,

g 0, 1 oder 2,

L 0 oder 1,

m 0, 1, 2, 3 oder 4,

p 0 oder, falls m ungleich 0 ist oder falls gleichzeitig L und m = 0 sind, 0 oder 1 und

X O oder falls m ungleich 0 ist oder gleichzeitig L, m und p = 0 sind oder falls gleichzeitig m und p = 0 und n ungleich 0 sind, O, S oder N-$R^3$, wobei $R^3$ H, $C_1$-$C_4$-Alkyl, Phenyl oder Benzyl bedeutet, und

$R^2$ unabhängig voneinander $C_1$-$C_4$-Alkyl, $C_1$-$C_4$-Alkoxy, F, Cl, Br, J, $SCH_3$, CO-$C_6H_5$, $CF_3$, $COOCH_3$, $COOC_2H_5$ oder $NO_2$ und

n 0, 1 oder 2 oder, falls n=2 ist,

$R^2$ zusätzlich eine -CH=CH-CH=CH-Gruppe , die mit dem Phenylring zusammen einen Naphthyl-Rest bildet, oder,

falls gleichzeitig p = 0 ist und n = 1 ist,

$R^2$ zusätzlich eine unsubstituierte oder im Phenylrest bis zu 2 gleiche oder verschiedene Substituenten tragende Phenoxygruppe darstellt, wobei die Substituenten F, Cl, Br, $OCH_3$, $CH_3$ oder $C_2H_5$ bedeuten,

sowie von deren physiologisch verträglichen Säureadditionssalzen, dadurch gekennzeichnet, daß man

A) eine Verbindung der Formel II,

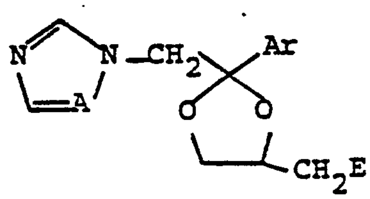

II,

in der

A und Ar die zu Formel I angegebenen Bedeutungen haben und

E Cl, Br, J oder Acyloxy, wie Acetoxy, Trifluoracetyloxy, Benzoyloxy, Nitrobenzoyloxy, $C_1$-$C_3$-Alkylsulfonyloxy wie Methansulfonyloxy, oder Arylsulfonyloxy, wie Benzol-, Nitrobenzol-, Brombenzol- oder Toluolsulfonyloxy, bedeutet,

mit einer Verbindung der Formel III,

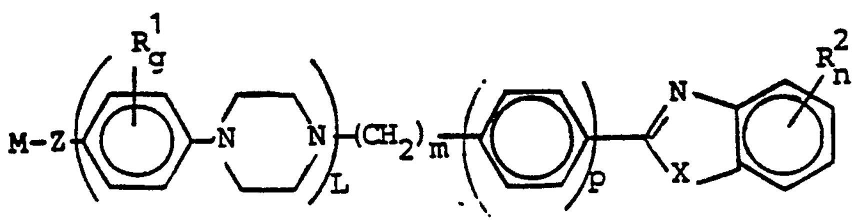

III,

in der

Z O oder,

falls gleichzeitig L, m und p Null bedeuten, S bedeutet und

M H, ein Alkali- oder Erdalkalimetall, insbesondere Li, Na oder K, oder $NH_4$ bedeutet und

$R^1$, g, L, m,p, X, $R^2$ und n die zu Formel I angegebenen Bedeutungen haben, umsetzt, oder daß man

B) eine Verbindung der Formel IV,

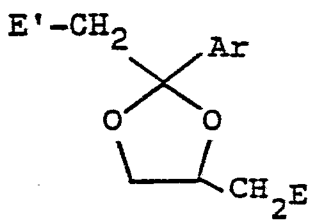

IV

in der

Ar die zu Formel I und E und E' die zu Formel II für E angegebenen Bedeutungen haben,

zunächst mit einer Verbindung der Formel III

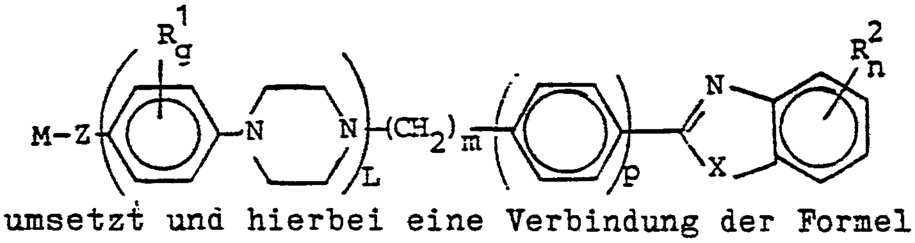

III

umsetzt und hierbei eine Verbindung der Formel V herstellt,

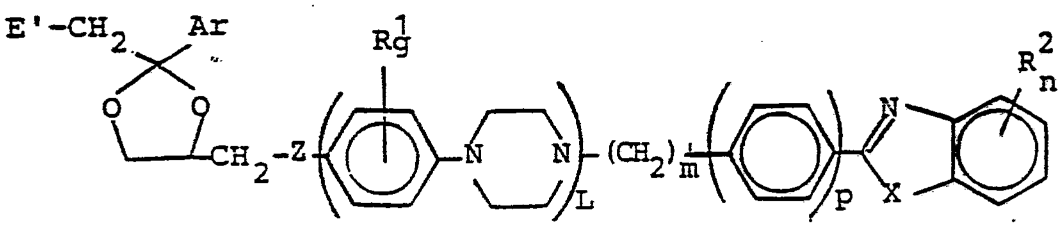

V

in der Z O oder, falls gleichzeitig L, m und p Null bedeuten, S bedeutet, und

Ar, $R^1$, g, L, m, p, X, $R^2$ und n die zu Formel I und E' die zu Formel II für E angegebenen Bedeutungen haben, und anschließend eine Verbindung der Formel V mit einer Verbindung der Formel VI umsetzt,

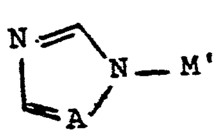

VI

in der

A CH oder N und

M' H, ein Alkali- oder Erdalkalimetall oder $Si(CH_3)_3$ bedeutet,

oder daß man

C) eine Verbindung der Formel VII,

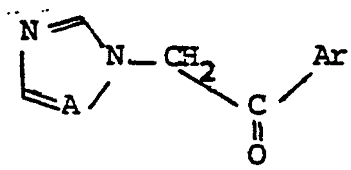

VII

in der A und Ar die zu Formel I angegebenen Bedeutungen haben, mit einem 1,2-Diol der Formel VIII

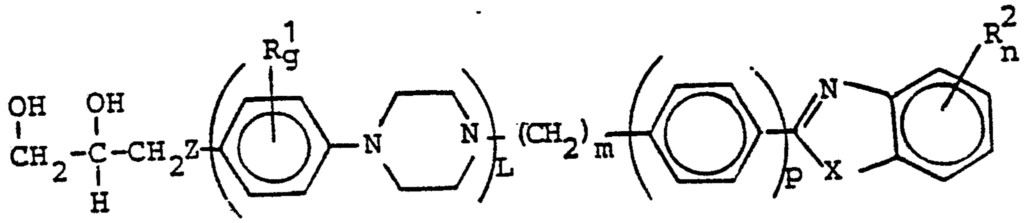

VIII

in der Z, $R^1$, g, L, m, p, X, $R^2$ und n die zu Formel I angegebenen Bedeutungen haben, umsetzt,

oder daß man

D) eine Verbindung der Formel IX,

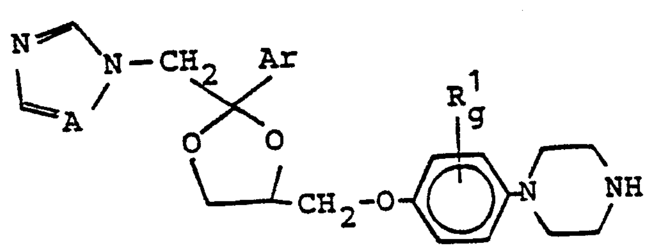

IX

in der A, Ar, $R^1$ und g die zu Formel I angegebenen Bedeutungen haben, oder ein Salz dieser Verbindung, entweder mit einer Verbindung der Formel X,

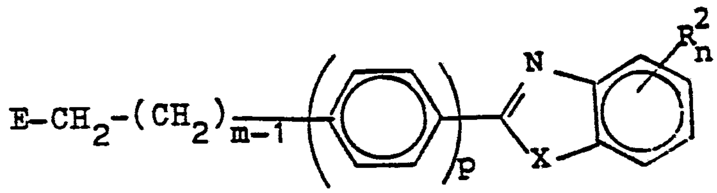

X

in der E Cl, Br, J, Acyloxy, wie Acetyloxy, Trifluoracetyloxy, Benzoyloxy, Nitrobenzoyloxy, $C_1$-$C_3$-Alkylsulfonyloxy, wie Methansulfonyloxy, oder Arylsulfonyloxy, wie Benzol-, Nitrobenzol-, Brombenzol- oder Toluolsulfonyloxy, und

m 1, 2, 3 oder 4 bedeutet, und

p, X, $R^2$ und n die zu Formel I angegebenen Bedeutungen haben,

oder mit einer Verbindung der Formel XI,

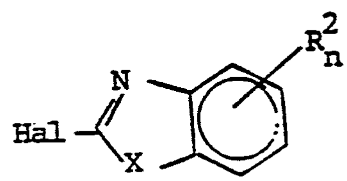

XI

in der

Hal Cl, Br, oder J, vorzugsweise Cl,

X O, oder,
falls n ungleich O ist, O, S oder $NR^3$, wobei $R^3$ H, $C_1$-$C_4$-Alkyl, Phenyl oder Benzyl bedeutet, bedeuten und

$R^2$ und n die zu Formel I angegebenen Bedeutungen haben, umsetzt, oder daß man

E) eine Verbindung der Formel IIa,

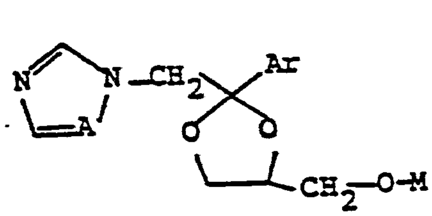

IIa

in der A und Ar die zu Formel I angegebenen Bedeutungen haben und

M H, ein Alkali- oder Erdalkalimetall, insbesondere Li, Na oder K bedeutet,

mit einer Verbindung der Formel X, in der $E$, $m$, $p$, $X$, $R^2$ und $n$ die zu Formel X angegebenen Bedeutungen haben, unter Bildung einer Verbindung der Formel I, in der Z O bedeutet und A, Ar, $R^1$, $g$, $m$, $p$, $X$, $R^2$ und $n$ die angegebenen Bedeutungen haben und L = O bedeutet, umsetzt, oder daß man

F) eine Verbindung der Formel IIa,

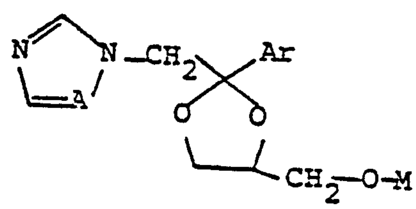

IIa

in der A und Ar die zu Formel I angegebenen Bedeutungen haben und

M H, ein Alkali- oder Erdalkalimetall, insbesondere Li, Na oder K, bedeutet,

mit einer Verbindung der Formel XI,

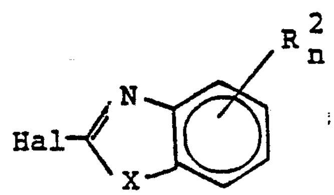

XI

in der

Hal Cl, Br oder J, vorzugsweise Cl, bedeutet und $X$, $R^2$ und $n$ die zu Formel I angegebenen Bedeutungen haben, unter Bildung einer Verbindung der Formel I, in der Z O bedeutet und A, Ar, $R^1$, $g$, $X$, $R^2$ und $n$ die angegebenen Bedeutungen haben und L, m und p gleichzeitig O bedeuten, umsetzt

und gegebenenfalls die nach Weg A)-F) erhaltenen Verbindungen der Formel I mit anorganischen oder organischen Säuren in ihre physiologisch verträglichen Salze überführt.

2. Verfahren zum Herstellen einer Verbindung der Formel I nach Anspruch 1, dadurch gekennzeichnet, daß mindestens einer der Substituenten und Indices bedeutet:

A CH oder N,
Ar eine durch 1 oder 2 F- oder Cl-Atome substituierte Phenylgruppe,
Z O oder,
falls gleichzeitig L, m und p Null bedeuten,
O oder S,
$R^1$ $CH_3$ oder $C_2H_5$,
g O oder 2,
L O oder 1,
m O, 1, 2 oder 3,
p O oder, falls m = 1 ist, O oder 1,
X O, oder
falls m = 1, 2 oder 3 ist, oder falls gleichzeitig L und m = O sind, oder falls gleichzeitig m = O ist und n = 1 oder 2 ist, O oder S,
$R^2$ unabhängig voneinander $C_1$-$C_4$-n-Alkyl, $C_1$-$C_4$-Alkoxy, F, Cl, Br oder, falls p = O und n = 1 ist, $OC_6H_5$ und
n O, 1 oder 2.

3. Verfahren zum Herstellen einer Verbindung der Formel I nach Anspruch 1, dadurch gekennzeichnet, daß mindestens einer der Substituenten und Indices bedeutet:

A CH oder N,
Ar 2,4-Dichlorphenyl,
Z O,
$R^1$ $CH_3$,

g 0 oder 2,
L 0 oder 1,
m 0, 1, 2 oder 3,
p 0 oder, falls m = 1 ist, 0 oder 1,
X 0 oder,
falls m = 1, 2 oder 3 ist oder falls gleichzeitig L und m = 0 sind, 0 oder S,
$R^2$ unabhängig voneinander $CH_3$, $C_2H_5$, $C_1$-$C_4$-Alkoxy, F, Cl oder Br und
n 0, 1 oder 2.

4. Verfahren zum Herstellen einer Verbindung der Formel I nach Anspruch 1, dadurch gekennzeichnet, daß gleichzeitig L, m und p Null oder L Null und gleichzeitig m und p 1 und jeweils X O oder S und n Null oder 1 bedeuten und $R^2$ $CH_3$, $C_2H_5$, $C_1$-$C_4$-Alkoxy, F, Cl oder Br bedeuten.

5. Verfahren zum Herstellen einer Verbindung I nach Anspruch 1, dadurch gekennzeichnet, daß der Azoylmethylrest und die 4-ständige Piperazinophenoxymethylgruppe am Dioxolanring in cis-Stellung stehen.

6. Verfahren zum Herstellen einer Verbindung der Formel IIIa

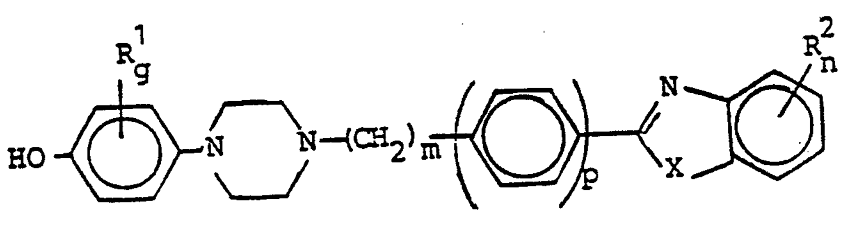

IIIa

in der bedeuten:
$R^1$ $C_1$-$C_3$-Alkyl, F oder Cl,
g 0, 1 oder 2,
m 0, 1, 2, 3 oder 4,
p 0 oder
falls m ungleich 0 ist, 0 oder 1 und

X O oder, falls m ungleich O ist oder falls gleichzeitig m und p=O und n ungleich O sind, O, S oder N-$R^3$, wobei

$R^3$ H, $C_1$-$C_4$-Alkyl, Phenyl oder Benzyl bedeutet, und

$R^2$ unabhängig voneinander $C_1$-$C_4$-Alkyl, $C_1$-$C_4$-Alkoxy, F, Cl, Br, J, $SCH_3$, CO-$C_6H_5$, $CF_3$ oder $NO_2$ und

n O, 1 oder 2

oder, falls n = 2 ist,

$R^2$ zusätzlich eine -CH=CH-CH=CH-Gruppe, die mit dem Phenylring zusammen einen Naphthyl-Rest bildet,

oder, falls p = O ist und n = 1 ist,

$R^2$ zusätzlich eine unsubstituierte oder im Phenylrest bis zu 2 gleiche oder verschiedene Substituenten tragende Phenoxygruppe, wobei die Substituenten F, Cl, Br, $OCH_3$, $CH_3$ oder $C_2H_5$ bedeuten,

sowie von deren Säureadditionssalze, dadurch gekennzeichnet, daß man eine Verbindung der Formel XVI,

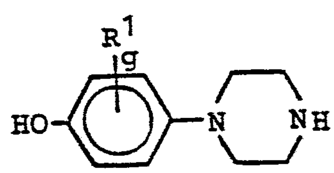

XVI

in der $R^1$ und g die zu Formel IIIa angegebenen Bedeutungen haben, oder ein Salz dieser Verbindung, entweder mit einer Verbindung der Formel X,

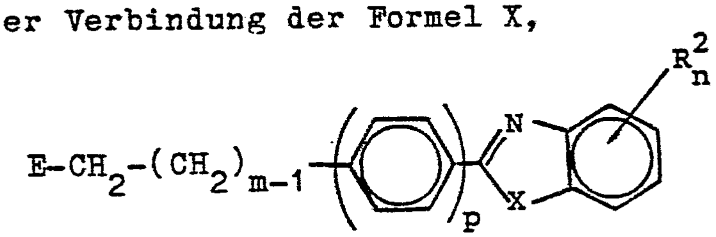

X

in der

E Cl, Br, J, Acyloxy, wie Acetoxy, Trifluoracetoxy, Benzoyloxy, Nitrobenzoyloxy, $C_1$-$C_3$-Alkylsulfonyloxy, wie Methansulfonyloxy, oder Arylsulfonyloxy, wie Benzol-, Nitrobenzol-, Brombenzol- oder Toluolsulfonyloxy und

m 1, 2, 3 oder 4 bedeutet, und

p, X, $R^2$ und n die zu Formel IIIa angegebenen Bedeutungen haben,

oder mit einer Verbindung der Formel XI,

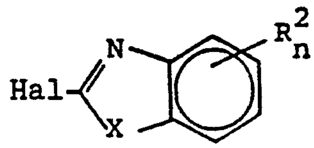

XI

in der

Hal Cl, Br oder J, vorzugsweise Cl,

X O, oder falls n ungleich Null ist, O, S oder $N-R^3$, wobei $R^3$ H, $C_1-C_4$-Alkyl, Phenyl oder Benzyl bedeutet, bedeuten und

$R^2$ und n die zu Formel IIIa angegebenen Bedeutungen haben, umsetzt,

und gegebenenfalls die erhaltenen Verbindungen der Formel IIIa mit anorganischen oder organischen Säuren in ihre Säureadditionssalze überführt.

7. Verwendung einer Verbindung der Formel I nach Anspruch 1 als Antimykotikum.

8. Verwendung einer Verbindung der Formel I nach Anspruch 1 zur Herstellung eines antimykotisch wirkenden Arzneimittels.

9. Verfahren zum Behandeln von Mykosen, dadurch gekennzeichnet, daß man eine wirksame Menge einer Verbindung I nach Anspruch 1 zusammen mit pharmazeutisch geeigneten Trägerstoffen appliziert.